(19)

**Europäisches Patentamt**
**European Patent Office**
**Office européen des brevets**

(11) **EP 3 919 047 A1**

(12) **EUROPEAN PATENT APPLICATION**

(43) Date of publication:
**08.12.2021 Bulletin 2021/49**

(21) Application number: **20178029.3**

(22) Date of filing: **03.06.2020**

(51) Int Cl.:
*A61K 9/00* (2006.01)  *A61K 9/107* (2006.01)
*A61K 47/02* (2006.01)  *A61K 47/12* (2006.01)
*A61K 47/14* (2017.01)  *A61K 47/26* (2006.01)
*A61K 47/36* (2006.01)  *A61P 27/04* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **AZAD Pharma AG**
**8200 Schaffhausen (CH)**

(72) Inventors:
- CARLI, Fabio
  **34127 Trieste (IT)**
- CHIELLINI, Elisabetta
  **34127 Trieste (IT)**
- BARONIAN, Mihran
  **3125 Toffen (CH)**

(74) Representative: **Weickmann & Weickmann PartmbB**
**Postfach 860 820**
**81635 München (DE)**

(54) **MICROEMULSION FOR THE TREATMENT OF DRY EYE SYNDROME**

(57) The present invention refers to a microemulsion, particularly suitable for ophthalmic applications such as treatment of dry eye syndrome, which comprises at least one oily component, an aqueous phase, at least one non-ionic surfactant, at least one polysaccharide, a salt or a derivative thereof, and at least one cross-linking agent. Moreover, the present invention relates to a process of manufacturing a microemulsion according to the invention, as well as its use in ophthalmic applications or as a carrier.

EP 3 919 047 A1

**Description**

The present invention refers to a microemulsion, particularly suitable for ophthalmic applications such as treatment of dry eye syndrome, which comprises at least one oily component, an aqueous phase, at least one non-ionic surfactant, at least one polysaccharide, a salt or a derivative thereof, and at least one cross-linking agent. Moreover, the present invention relates to a process of manufacturing a microemulsion according to the invention, as well as its use in ophthalmic applications or as a carrier.

Dry eye syndrome (DES), associated with symptoms like eye irritation, dryness, discharge, foreign body sensation and blurred vision, is affecting up to 34% of the world's population. Without adequate treatment, a chronic DES condition might even result in damage of the ocular surface with punctate epithelial erosion and conjunctival scarring.

Minor symptoms of DES can be treated by an application of artificial tears, such as oil-in-water emulsions, ameliorating e.g. foreign body sensation. However, cationic surfactants and/or preservatives present in those compositions are suspected to cause eye irritation themselves.

A more severe progression of DES might be treated by application of compositions comprising active agents such as cyclosporine A, which is an immunosuppressant and leads to increased production of tear fluid, or lifitegrast, which is an integrin antagonist and reduces eye inflammation. However, long-term application of drugs may cause unintended side effects and results in low patient compliance.

Recently, microemulsions comprising micronized oil droplets being dispersed in an aqueous medium were found to be promising formulations for ophthalmic applications, such as treatment of DES.

EP 3 409 268 discloses an ophthalmic oil-in-water emulsion comprising ophthalmically acceptable omega-3 fatty acid-containing oil, a hydrophilic surfactant, a hydrophobic non-co-block surfactant and water, wherein said oil-in-water emulsion composition has an average particle size less than 1 $\mu$m in diameter. The hydrophilic surfactant can be any surfactant suitable for use in pharmaceutical compositions, i.e. an anionic, cationic, zwitterionic or non-ionic surfactant. Further ingredients may be active agents such as cyclosporine A, and preservatives such as polyhexamethylene biguanide (PHMB).

WO 2019/036625 discloses a composition for treating an eye of an mammal, comprising water and a therapeutically efficient concentration of a hydrophobic component selected from various oils and derivatives thereof. To stabilize the composition, it includes an effective amount of a preservative component, such as benzalkonium chloride (BAK).

Considering the common application of (potentially) critical ingredients in ophthalmic compositions, such as ionic surfactants, irritating preservatives or active agents, there is an urgent need for the provision of improved pharmaceutical formulations with minimized or even avoided presence of harmful ingredients, while providing good DES treatment properties.

Thus, it was an object of the present invention to provide a pharmaceutically acceptable composition which is particularly suitable for treating dry eye syndrome and overcomes the above discussed issues.

It was surprisingly found that a microemulsion comprising at least one oily component, an aqueous phase, at least one non-ionic surfactant, at least one polysaccharide, a salt or a derivative thereof, and at least one cross-linking agent exerts strong activity against DES, while avoiding a disadvantageous application of e.g. ionic compounds such as ionic surfactants, or eye-irritating active agents and preservatives. As described in more detail below, the inventive microemulsions were found to have a high shelf-life, improved wetting and coverage properties, as well as good ocular tolerability.

Thus, a first aspect of the present invention is directed to a microemulsion comprising

i. at least one oily component,
ii. an aqueous phase,
iii. at least one non-ionic surfactant,
iv. at least one polysaccharide, a salt or a derivative thereof, and
v. at least one cross-linking agent.

The at least one oily component (i) is insoluble in water. As used herein, the term *"insoluble in water"* means a solubility in water of less than 2.0 g/l, preferably less than 0.5 g/l, more preferably 0.2 g/l at 20°C.

In a preferred embodiment, the at least one oily component is selected from the group consisting of fatty acids and fatty acid esters, particularly unsaturated fatty acids and fatty acid esters, and mixtures thereof.

In a more preferred embodiment, the at least one oily component is selected from the group consisting of

- fatty acid triglycerides, such as glyceryl tricaprate, glyceryl trilaurate, glyceryl trilinoleate, natural occurring oils deriving from plants or animals such as olive oil, sesame oil, sunflower oil, soybean oil, castor oil, ricinus oil, corn oil and fish oil, but not limited thereto;

- fatty acid diglycerides, such as propylene glycol caprylate, propylene glycol caprate, diolein, dilinoleate, but not limited thereto;

- fatty acid monoglycerides such as monoolein, monopalmitolein, monomyristolein, but not limited thereto;

- fatty acid esters of monohydric alcohol such as ethyl oleate, isopropyl myristate, isopropyl palmitate, but not limited thereto;

- fatty acids such as oleic acid, linoleic acid, fish oil, but not limited thereto;

and mixtures thereof.

*"Fatty acid"* as used herein means a straight or branched chain, saturated or unsaturated, optionally substituted, hydrocarbon with at least 8, preferably at least 10, preferably at least 12, more preferably at least 14, even more preferably at least 16 and most preferably at least 20 carbon atoms and preferably up to 34 carbon atoms having a carboxylic acid functional group.

As used herein, the term *"unsaturated'* means an at least monounsaturated compound, i.e. a compound having at least one multiple bond, such as a double bond or a triple bond, preferably a double bond.

In a preferred embodiment, *"monohydric alcohol"* is a $C_{1-8}$ monohydric alcohol, preferably a $C_{1-5}$ monohydric alcohol, such as for example methanol, ethanol, propanol or isopropanol. In particular, monohydric alcohol is methanol, ethanol, propanol or isopropanol, even more preferably methanol, ethanol and isopropanol, and most preferably ethanol and isopropanol.

As used herein the term *"fatty acid (mono-, di- or tri-)glycerides"* refers to compounds wherein one, two or three hydroxy groups of glycerol are esterified or/and etherified with optionally hydrogenated synthetic or naturally occurring fatty acid(s) whereas possibly remaining hydroxy groups of the glycerol (if any) rest unreacted.

In another embodiment, the at least one oily component (i) is selected from the group consisting of fatty acid triglycerides, fatty acids, fatty acid esters of monohydric alcohols and mixtures thereof, preferably unsaturated fatty acid triglycerides, fatty acids, fatty acid esters of monohydric alcohols and mixtures thereof.

In another embodiment, the at least one oily component (i) is selected from the group consisting of fatty acids, fatty acid esters of monohydric alcohols and mixtures thereof, preferably unsaturated fatty acids, fatty acid esters of monohydric alcohols and mixtures thereof.

In another embodiment, the at least one oily component (i) is selected from the group consisting of fatty acid esters of monohydric alcohol, fatty acid triglycerides and mixtures thereof, preferably unsaturated fatty acid esters of monohydric alcohol, fatty acid triglycerides and mixtures thereof.

In another embodiment, the at least one oily component (i) is selected from the group consisting of fatty acids, fatty acid triglycerides and mixtures thereof, preferably unsaturated fatty acids, fatty acid triglycerides and mixtures thereof.

In another embodiment, the at least one oily component (i) is at least one fatty acid ester of a monohydric alcohol, preferably at least one unsaturated fatty acid ester of a monohydric alcohol.

In one embodiment the oily component is free of fatty acid triglycerides or/and fatty acid diglycerides or/and fatty acid monoglycerides. In a preferred embodiment, the oily component is free of fatty acid mono- or diglycerides. In another preferred embodiment, the oily component is free of fatty acid triglycerides.

Preferably, the oily component (i) is selected from the group consisting of ethyl oleate, oleic acid, ricinus oil, corn oil, or mixtures thereof.

In a preferred embodiment, the oily component contains one, two, three or four different oily compounds, in particular one or two, such as ethyl oleate and/or oleic acid.

The oily component (i) is preferably present in an amount of 1.0 - 10.0 wt.-%, more preferably of 2.0 - 8.0 wt.-%, and even more preferably of 3.0 - 7.0 wt.-%, based on the total weight of the microemulsion.

The presence of at least one oily component according to the invention allows for the provision of an improved ophthalmic composition. For example, the oily component is advantageous in terms of tear film layer reconstruction, a reduction of water evaporation from the eye and a lubrication of the ocular surface, which is particularly advantageous in the treatment of dry eye syndrome.

The aqueous phase (component (ii)) according to the invention comprises water, preferably a pharmaceutically acceptable type of water, such as sterile water, deionized water, or water for injectables according to Ph. Eur.

The aqueous phase (ii) may further comprise water soluble formulation aids known in the art, which are e.g. suitable for adjusting pH value, viscosity, stability etc. Examples for formulation aids are buffer agents such as potassium phosphate, sodium borate, or sodium gluconate, isotonic agents such as sucrose, sodium chloride, or potassium chloride, viscosity-increasing compounds such as polyvinylpyrrolidone, antimicrobial preservatives such as stabilized oxychloro complex (SOC), stabilizers such as emulsifiers, or mixtures thereof, but are not limited thereto.

The aqueous phase may be present in an amount of 50.0 - 98.0 wt.-%, preferably 60.0-95.0 wt.-%, and more preferably 70.0 - 90.0 wt.-% based on the total weight of the microemulsion.

The formulation aid may be present in an amount of from 0 to 30 wt.-%, preferably from 0 to 20 wt.-%, and more preferably from 0 to 15 wt.-% based on the total amount of the aqueous phase.

The microemulsion further comprises at least one non-ionic surfactant (component (iii)), which particularly differs from the oily component (i). A *"non-ionic surfactant"* as used herein is a surfactant which does not exhibit an ionic charge under conditions under which it is used.

Suitable non-ionic surfactants are e.g. alkylglucosides, polyoxyalkylene alkyl ethers, polyoxyalkylene alkyl phenols, polyoxyalkylene fatty acid esters, polyoxyalkylene glycerol fatty acid esters, polyoxyalkylene sorbitan fatty acid esters, polyoxyalkylene blockcopolymers, polyglycerol fatty acid esters, polyoxyalkylene glycerides, polyoxyalkylene sterols, polyoxyalkylene vegetable oils, polyoxyalkylene hydrogenated vegetable oils, polyglycerol ether, polyoxyalkylene glycerol ester, polyvinylalcohol, and mixtures thereof.

In a preferred embodiment, the at least one non-ionic surfactant may be selected from the group consisting of polyoxyethylene alkyl ethers, polyoxyethylene sorbitane fatty acid esters, polyoxyethylene fatty acid esters, polyoxyethylene glycerol fatty acid esters, polyoxyethylene polyoxypropylene block copolymers, polyglycerol fatty acid esters, polyoxyethylene sterols, polyoxyethylene, but are not limited thereto.

In another preferred embodiment, the microemulsion contains at least one non-ionic surfactants being sterically voluminous. Examples for voluminous non-ionic surfactants are polyoxyethylene-polyoxypropylene block copolymers, polyvinylalcohol, polyoxyethylene sorbitane fatty acid esters, polyoxyethylene alkyl ethers, and polyoxyethylene fatty acid esters, in particular polyoxyethylene - polyoxypropylene block copolymers, polyoxyethylene alkyl ethers, polyoxyethylene fatty acid esters, and polyoxyethylene sorbitane fatty acid esters, more preferably polyoxyethylene sorbitane fatty acid esters.

In a preferred embodiment, component (iii) is free of fatty acid monoglycerides or/and fatty acid diglycerides as defined above.

The total amount of the at least one non-ionic surfactant (iii) is preferably in the range of 0.40 - 10.00 wt.-%, more preferably in the range of 0.50 - 5.0 wt.-%, and even more preferably in the range of 1.0 - 5.0 wt.-%, based on the total weight of the microemulsion.

In one embodiment, the microemulsion according to the invention comprises at least two structurally different non-ionic surfactants which are different from the oily component (i). By combining two structurally different non-ionic surfactants, the interaction between the oily component (i) and the aqueous phase (ii) leads to a fine dispersion of the oil in the water phase. Particularly, the microemulsion comprises 2, 3 or 4, preferably two non-ionic surfactants.

Preferably, the microemulsion comprises at least two, particularly two, non-ionic surfactants selected from the combinations polyoxyethylene (20) sorbitan monooleate (Tween 80®) / polyoxyethylene (20) sorbitan monolaurate (Tween 20®), polyoxyethylene (20) sorbitan monooleate (Tween 80®) / polyoxyl (2) cetylether (Brij 52®), polyoxyl (2) cethylether (Brij 52®) / polyoxyl (20) cethylether (Brij 58®) and polyoxyl (20) cethylether (Brij 58®) / polyoxyl (10) cethylether (Brij 56®) as first / second non-ionic surfactant, respectively.

The ratio of first non-ionic surfactant to second non-ionic surfactant may be 10:1 - 1:1, particularly 2:1 - 1:1. In a preferred embodiment, the microemulsion according to the invention comprises Tween 80® (first non-ionic surfactant) and Tween 20® (second non-ionic surfactant) in a ratio of 2:1 - 1:1.

The microemulsion further comprises at least one polysaccharide, a salt or a derivative thereof (component (iv)), which may be present in an amount of 0.001 to 0.100 wt.-%, preferably 0.020 to 0.050 wt.-%, based on the total weight of the microemulsion.

A *"polysaccharide"* as used herein is a carbohydrate polymer comprising monosaccharide repeating units, which are interconnected by glycosidic linkages. A polysaccharide can have one type of repeating unit, i.e. a homopolysaccharide, or two or more types of repeating units, i.e. a heteropolysaccharide. The number of repeating units per polysaccharide molecule may be at least 10, such as 40-3000 or 200-2500. Particularly, a polysaccharide may comprise (modified) glucose, fructose, and/or glyceraldehyde repeating units.

In one embodiment, component (iv) is a polysaccharide salt. A polysaccharide salt comprises anionic, cationic, or zwitterionic polysaccharides and at least one type of counter ion. Suitable counter ions may be selected from alkali and/or earth alkali metal ions, such as $Na^+$, $K^+$, $Ca^{2+}$, or $Mg^{2+}$, for polysaccharides having an anionic charge, and halides such as $Cl^-$ or Br, for polysaccharides having a cationic charge.

In one embodiment, component (iv) is a polysaccharide derivative. A polysaccharide derivative according to the invention is a chemically modified polysaccharide such as an alkylated, hydroxyalkylated, sulfonated, nitrated, carboxyalkylated or/and xanthogenated polysaccharide. By modification of polysaccharides, compounds whose profile of properties are customized for the respective application, e.g. with regard to water solubility, are provided.

In a preferred embodiment, component (iv) is selected from hyaluronic acid, pectin, cellulose, cellulose derivatives e.g. carboxymethyl cellulose, alginic acid, carrageenan, chitosan, a salt thereof or a mixture thereof, preferably a pharmaceutically acceptable salt of hyaluronic acid, particularly sodium hyaluronate.

The presence of a polysaccharide, a salt or a derivative thereof may be advantageous in terms of the hydrating properties of the microemulsion, e.g. for hydration of the eye surface in the treatment of dry eye syndrome.

The microemulsion further comprises at least one cross-linking agent (component (v)). Component (v) may be present in an amount of 0.005 to 0.100 wt.-%, preferably 0.01-0.075 wt.-%, more preferably 0.01 - 0.050 wt.-% based on the total weight of the microemulsion.

A cross-inking agent (v) according to the invention is particularly capable of crosslinking component (iv). Component (v) may be selected from physical cross-linking agents such as ionic cross-linking agents, and chemical cross-linking agents such as radical cross-linking agents, or cross-linking agents inducing a condensation reaction or an addition reaction.

In one embodiment, component (v) may have at least one carboxyl group or a salt thereof, and is preferably polycarboxylic acid e.g. citric acid, ethylenediaminetetraacetic acid (EDTA), maleic acid, a salt thereof or a mixture thereof.

In another embodiment, component (v) may be a cationic cross-linking agent such a multivalent metal ion, particularly $Ca^{2+}$ or $Mg^{2+}$, a salt thereof or a mixture thereof.

The weight ratio of component (iv) to component (v) may be 1:1 - 1:20, preferably 1:1-1:2. In a preferred embodiment, the microemulsion according to the invention comprises hyaluronic acid or a salt thereof (component (iv)) and citric acid or a salt thereof (component (v)) in a weight ratio of 1:1 - 1:2.

Moreover, the microemulsion according to the invention may comprise a co-surfactant (component (vi)). The co-surfactant differs from the non-ionic surfactant (iii) and the oily component (i) and may be selected from, for example, non-ionic surfactants, monoalcohols, polyols, and mixtures thereof, particularly monoalcohols, polyols and mixtures thereof.

The co-surfactant accumulates - as the surfactant (iii) - at the interface between the oily component (i) and the aqueous phase (ii). By incorporating at least one co-surfactant, the interface layer is packed more densely and is thus more water-repelling, which reduces the possibility of molecular aggregates at the interface.

Suitable co-surfactants may be selected from the group consisting of polyglycerol ester with fatty acids, polyoxyalkylated alkylether, diols, monohydric alcohols, polyoxyalkylated (hydrogenated) oil, sugar alcohols and mixtures thereof, but are not limited thereto.

In a preferred embodiment, the co-surfactant is selected from the group consisting of polyglycerol-6-dioleate, ethanol, n-propanol, 1,2-propyleneglycol, polyoxyethylene hydrogenated castor oil, sorbitol, glycerol, but is not limited thereto. More preferably, the co-surfactant is selected from ethanol, 1,2-propylene glycol, sorbitol, glycerol and mixtures thereof.

In a preferred embodiment the co-surfactant is free of fatty acid mono- or/and diglycerides.

The amount of co-surfactant may range from 1.00 to 5.00 wt.-%, preferably from 2.00 to 5.00 wt.-%, based on the total weight of the microemulsion.

In a preferred embodiment, the microemulsion according to the invention comprises:

as component (i) at least one fatty acid ester of monohydric alcohol, in particular ethyl oleate,
as component (ii) a pharmaceutically-acceptable water,
as component (iii) at least one polyoxyalkylene sorbitan fatty acid ester, in particular polyoxyethylene sorbitane monooleate (Tween 80®) and polyoxyethylene sorbitane laurate (Tween 20®),
as component (iv) hyaluronic acid or a salt thereof, in particular sodium hyaluronate,
as component (v) citric acid or a salt thereof, particularly calcium citrate, and
optionally as co-surfactant (vi) sorbitol.

In a particularly preferred embodiment, component (i) is ethyl oleate, component (iii) is a mixture of polyoxyethylene sorbitane monooleate (Tween 80®) and polyoxyethylene sorbitane monolaurate (Tween 20®), and component (v) is calcium citrate.

Preferably, components (i) to (vi) are present in the following amounts based on the total weight of the emulsion:

1.0 - 10.0 wt.-% component (i), preferably ethyl oleate,
50.0 - 98.0 wt.-% component (ii), preferably a pharmaceutically acceptable water,
0.40 - 10.00 wt.-% component (iii), preferably Tween 80® and Tween 20®,
0.001 - 0.050 wt.-%, preferably 0.020 - 0.050 wt.-%, component (iv), preferably hyaluronic acid or a salt thereof,
0.005 - 0.100 wt.-% component (v), preferably citric acid or a salt thereof, and
optionally 1.00 - 5.00 wt.-% component (vi), preferably sorbitol.

In a preferred embodiment, the weight ratio of component (ii) to component (i) is lower than 50:1, more preferably lower than 20:1, and even more preferably between 20:1 and 10:1.

Thus, in a preferred embodiment, the microemulsion according to the present invention is an oil-in-water microemulsion which means that individual droplets of the oily component are distributed within the continuous aqueous phase.

The mean average diameter of the oily component droplets may be in the range of 5 to 10,000 nm, preferably in the range of 10 to 2,000 nm, more preferably in the range of 50 to 1,000 nm (measured according to ISO/DIS 22412). The diameter also includes the non-ionic surfactants assembling at the interface oily component/aqueous phase.

The microemulsions according to the invention are highly stable, i.e. the droplet size remains unchanged over time, preferably over a period of 6 months, more preferably over a period of 8 weeks, at room temperature, i.e. 20 °C, and ambient pressure, i.e. 1 bar.

Without being bound to any theory, the advantageous microemulsion stability is related to the presence of the at least one polysaccharide (iv) and the at least one cross-linking agent (v) capable of cross-linking component (iv). By forming a polysaccharide-based polymer network in the aqueous phase of the microemulsion, components (iv) and (v) prevent the oil droplets from undergoing, for example, aggregation, coalescence, Oswald ripening, sedimentation and creaming. Thus, a microemulsion having and maintaining a homogeneous structure is provided.

Moreover, the inventive microemulsion is particularly free from potentially harmful ingredients such as preservatives, e.g. PHMB or BAK, and ionic surfactants, particularly cationic surfactants, causing eye irritation.

In one embodiment, the microemulsion according to the invention is free from a drug.

The term "*drug*" refers to a compound or a mixture of compounds which is intended for use in or on the human or animal body having properties for treating, alleviating and/or preventing human and/or animal diseases or medical conditions. A drug can be used in or on the human or animal body or administered to a human or an animal in order to either restore, correct or influence physiological functions through pharmacological, immunological or metabolic action; or serve as a basis for a medical diagnosis.

Particularly, the inventive microemulsion is free from drugs to treat, alleviate and/or prevent a disease or condition of the eye, such as dry eye. For example, the microemulsion may be free from cyclosporine, lifitegrast, rituximab, tocilizumab, rivoglitazone, mapracorat, resolving, tasoticinib, tofacitinib, voclosporin, thymosin, ecabet, rimexolone, rebamipide, diquafosol, bromfenac, and/or dexamethasone.

In another embodiment, the inventive microemulsion is particularly free from drugs to treat, alleviate and/or prevent a disease or condition of eye infection either of bacterial or viral origin. For example, the microemulsion may be free from ciprofloxacin, ofloxacin, neomycin, tobramycin, ganciclovir, famciclovir, valganciclovir, acyclovir, and/or hexamidine.

In another embodiment, the inventive microemulsion is particularly free from drugs to treat, alleviate and/or prevent a disease or condition of inflammation. For example, the microemulsion may be free from dexamethasone, fluorometholon, hydrocortisone, prednisolone, ketorolac, diclofenac, indomethacin, ketorolac, nepafenac, and/or bromfenac.

In another embodiment, the inventive microemulsion is particularly free from drugs to treat, alleviate and/or prevent a disease or condition of glaucoma. For example, the microemulsion may be free from brinzolamide, acetazolamide, dorzolamide, brimonidine, carteolol, betaxol, timolol, levobunol, latanoprost, tafluprost, bimatoprost, and/or travoprost.

In another embodiment, the inventive microemulsion is particularly free from drugs to induce pupil wide opening and to treat inflammation conditions of specific eye components e.g. iritis, cyclitis, cyclopegia. For example, the microemulsion may be free from atropine, cyclopentolate, tropicamid, and/or scopolamine.

In another embodiment, the inventive microemulsion is particularly free from drugs to treat, alleviate and/or prevent a disease or condition of eye congestion. For example, the microemulsion may be free from tetryzoline, naphazoline, antazoline.

In another embodiment, the inventive microemulsion is particularly free from drugs to treat, alleviate and/or prevent a disease or condition of allergic eye disease . For example, the microemulsion may be free from azelastine, emedastin, levocabastin, olopatadine, ketotifen.

In another embodiment, the inventive microemulsion is particularly free from drugs to treat, alleviate and/or prevent a disease or condition of diabetic rethinopathy or agerelated macular disease. For example, the microemulsion may be free from aflibercept, ranibizumab, bevacizumab.

In another embodiment, the microemulsion according to the invention comprises at least one additional drug (component (vii)). Particularly, component (vii) is selected from the following non-exhaustive list: rituximab, tocilizumab, rivoglitazone, mapracorat, resolving, tasoticinib, tofacitinib, voclosporin, thymosin, ecabet, rimexolone, rebamipide, diquafosol, bromfenac, dexamethasone, ciprofloxacin, ofloxacin, neomycin, tobramycin, ganciclovir, famciclovir, valganciclovir, acyclovir, hexamidine, dexamethasone, fluorometholon, hydrocortisone, prednisolone, ketorolac, diclofenac, indomethacin, ketorolac, nepafenac, bromfenac, brinzolamide, acetazolamide, dorzolamide, brimonidine, carteolol, betaxol, timolol, levobunol, latanoprost, tafluprost, bimatoprost, travoprost, atropine, cyclopentolate, tropicamid, scopolamine, tetryzoline, naphazoline, antazoline, azelastine, emedastin, levocabastin, olopatadine, ketotifen, aflibercept, ranibizumab, and bevacizumab.

The at least one additional drug (vii) may be dissolved in the oily component (i). In another embodiment, the at least one additional drug (vii) may be dissolved in the aqueous phase (ii).

The amount of component (vii) may range from 0.001-10.0 wt.-%, preferably 0.001-1.0 wt.-%, based on the total weight of the microemulsion.

In a further aspect, the present invention refers to a process of manufacturing a microemulsion according to the invention, comprising the steps of

(a) preparing a mixture of components (i), (iii), (iv), (v), optionally (vi), and optionally (vii), and
(b) adding component (ii) to the mixture obtained in step (a) under stirring.

Preparing a mixture in step (a) is performed by conventional means known in the art such as with a magnetic stirrer, with a paddle stirrer, by hand shaking, or by using a homogenizer.

To the mixture obtained in step (a) the aqueous phase (component (ii)) is added under stirring, e.g. for 0.5 - 3 h. Addition of component (ii) can take place at once or in several portions.

In a preferred embodiment only little energy input is sufficient in order to obtain the stable microemulsion according to the invention. Preferably, it is sufficient to agitate the mixture in step (b) with a magnetic stirrer, a paddle stirrer or by hand shaking. Alternatively a homogenizer or a high energy homogenizer can be used.

In another aspect, the present invention refers to a microemulsion obtainable by a process as described above.

In still another aspect, the present invention refers to a pharmaceutical composition comprising a microemulsion according to the invention. Such pharmaceutical composition is particularly suitable for ophthalmic applications, preferably for the prevention, alleviation and/or treatment of dry eye syndrome.

Moreover, the present invention refers to a microemulsion or pharmaceutical composition according to the invention for use in ophthalmic applications, preferably for the prevention, alleviation and/or treatment of dry eye syndrome.

In still another aspect, the present invention refers to the use of a microemulsion as described herein as a carrier, in particular a drug carrier, such as an ophthalmic drug carrier. Potential drugs to be carried are e.g. as described herein.

The invention is further illustrated by the following Figures and Examples.

**Figure legends**

**Figure 1:** Percentage cell viability of human epithelium cells after single treatment relative to the negative control (NC).

**Figure 2:** Percentage cell viability of human epithelium cells after repeated treatment relative to the negative control (NC).

**Figure 3:** Histological analysis of *in vitro* dry eye models treated with different ophthalmic compositions (P1-P4) compared to the negative control (NC) and the positive dryness control.

**Figure 4:** Biomarker patterns of *in vitro* dry eye models treated with different ophthalmic compositions (P1 = Cationorm®, P2 = Artelac® Splash, P3 = Systane® UD, P4 = ME) during dryness stress induction for 36 h relative to the negative control (RQ = 1).

**Figure 5:** Biomarker patterns of *in vitro* dry eye models treated with different ophthalmic compositions (P1 = Cationorm®, P2 = Artelac® Splash, P3 = Systane® UD, P4 = ME) for 6 h after dryness stress induction for 36 h relative to the negative control (RQ = 1).

**Figure 6:** Biomarker patterns of *in vitro* dry eye models treated with different ophthalmic compositions (P1 = Cationorm®, P2 = Artelac® Splash, P3 = Systane® UD, P4 = ME) for 24 h after dryness stress induction for 36 h relative to the negative control (RQ = 1).

**Figure 7:** TEER analysis of *in vitro* dry eye models treated with different ophthalmic compositions (P1 = Cationorm®, P2 = Artelac® Splash, P3 = Systane® UD, P4 = ME) during dryness stress induction for 36 h compared to the negative control (NC) and the positive dryness control (PC).

**Figure 8:** TEER analysis of *in vitro* dry eye models treated with different ophthalmic compositions (P1 = Cationorm®, P2 = Artelac® Splash, P3 = Systane® UD, P4 = ME) for 6 h and 24 h after dryness stress induction for 36 h compared to the negative control (NC) and the positive dryness control (PC).

**Figure 9:** Histological analysis of a HCE negative control (corresponding to the reference morphology) and an *in vitro* dry eye model (positive control).

**Figure 10:** Histological analysis of *in vitro* dry eye models treated with different ophthalmic compositions (P1 = ME, P2 = Xiidra® Lifitegrast) compared to the positive dryness control.

**Figure 11:** Biomarker patterns of *in vitro* dry eye models after 40 h dry stress induction (PC 40h) and 24 h recovery (PC 40h+24h) relative to the negative control (NC 40h+24h, RQ = 1).

**Figure 12:** Biomarker patterns of *in vitro* dry eye models treated with different ophthalmic compositions (P1 = ME, P2 = Xiidra® Lifitegrast) for 24 h after dryness stress induction for 40 h relative to the positive control (PC 40h+24h, RQ = 1).

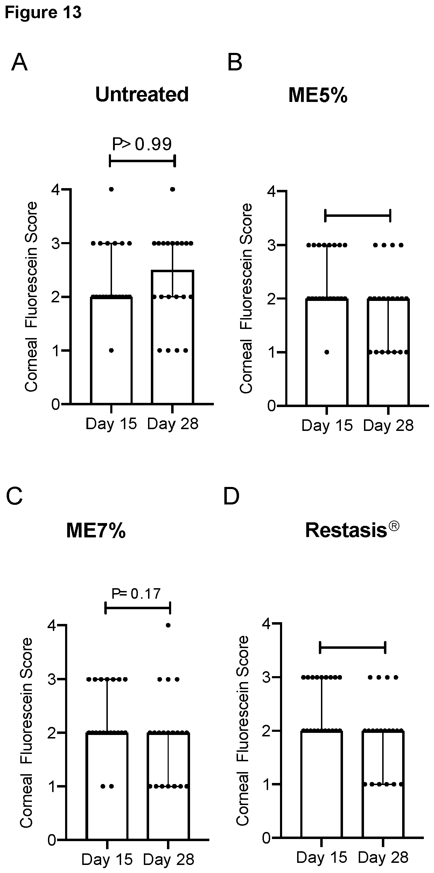

**Figure 13:** Corneal fluorescein staining effect. ME5% (A) resulted in improved corneal fluorescein staining on day 28 compared with day 15, similar to that observed by the reference compound (B). ME7% (C) showed a statistical trend towards improved corneal fluorescein scores. Corneal fluorescein staining in untreated eyes (D) was not significantly different between days 15 and 28. Data are presented as box/whisker plots, indicating the median (line), $25^{th}/75^{th}$ percentile (box) and the range (whiskers). Individual data points are depicted as filled circles. The number of eyes (n) was 20 per groups.

## Examples

### Example 1 - Microemulsion preparation

5.00 g of ethyl oleate, 1.00 g of Tween 80®, 0.50 g of Tween 20®, 4.10 g of d-sorbitol, 0.026 g of sodium hyaluronate, and 0.03 g of calcium citrate were provided in a reaction vessel and 1M aqueous NaOH was added to adjust the pH to a value of 6.8-7.4. Then, the compounds were mixed under stirring for 1-4 hours at room temperature.
Next, water for injectables was added gradually to the reaction vessel under stirring for 1-4 hours at room temperature.
The mixture obtained was sterilized using sterile filtration at a pore size of 0.22 $\mu$m.
The quantitative composition (%) of the resulting microemulsion is reported below.

### Composition of Example 1 (wt-%):

| | |
|---|---|
| ETHYL OLEATE (i) | 5.00 % |
| TWEEN 80® (iii) | 1.00 % |
| TWEEN 20® (iii) | 0.50 % |
| d-SORBITOL (vi) | 4.10 % |
| SODIUM HYALURONATE (iv) | 0.026 % |
| CALCIUM CITRATE (v) | 0.03 % |
| NaOH 1M | q.s. |
| WATER (ii) | ad 100 % |

### Example 2 - Microemulsion stability

The physical stability of different microemulsions was evaluated dependent on the concentration of cross-linking agent. Three microemulsions were prepared according to Example 1, whereas the concentration of calcium citrate was 0, 0.03 or 0.06 wt.-%.
The physical stability of these three microemulsions was evaluated using the Turbiscan™ apparatus. This apparatus determines the transmitted and backscattered light of a microemulsion placed in a vertical tube over the entire length of the tube from the bottom to the top. Movement of emulsified oil droplets towards the bottom (i.e. sedimentation) or to the top (i.e. creaming) leads to great variation of the TSI (Turbiscan Index) over time, which is derived from the sum of transmitted/backscattered light. That is, higher TSI values indicate a tendency of the microemulsion to separate.

The TSI was measured at different time points over 48 h after microemulsion (ME) preparation, in order to derive a rate of change of physical homogeneity in dependence of the calcium citrate concentration. Table 1 displays the calculated TSI change rates.

**TABLE 1**

| % calcium citrate in the ME | TSI Change Rate/h |
|---|---|
| 0 | 0.094 TSI/h |
| 0.03 | 0.018 TSI/h |
| 0.06 | 0.020 TSI/h |

The above data show that the addition of 0.03 wt.-% of calcium citrate leads to a large improvement of the physical stability of the microemulsion with a dramatic decrease of the migration of droplets compared to the microemulsion which is free from cross-linking agent (0% calcium citrate); the addition of 0.06 wt.-% of cross-linking agent does not exert any additional effect.

Example 3 - Contact angle measurement

A microemulsion composition was prepared according to Example 1 and transferred in a multidose eye drop container. Then, 10 drops of the microemulsion (ME) were erogated to a polymeric PE substrate one after another, without wiping of the container tip. Each drop was analysed with regard to its contact angle using a Wettabilty Tester Lorentzen-Wettre.

As comparative examples, the contact angles of the commercially available ophthalmic compositions for treatment of dry eye syndrome DROPSTAR®, HYALISTIL®, XILOIAL®, HYABAK®, and VISMED® were determined accordingly.

The contact angles are displayed in Table 2 (mean value and standard deviation S.D. out of 10 replicates).

**TABLE 2**

| Formulation | Mean | S.D. |
|---|---|---|
| ME | 38.3 | 5.8 |
| DROPSTAR® | 119.3 | 4.4 |
| HYALISTIL® | 114.3 | 4.9 |
| XILOIAL® | 118.2 | 4.6 |
| HYABAK® | 112.1 | 4.9 |
| VISMED ® | 115.1 | 4.3 |

A low contact angle corresponds to increased wettability and coverage properties of an ophthalmic composition when applied to the cornea surface. Thus, the microemulsion according to the invention (ME) clearly shows an improved efficacy with regard to wettability and coverage properties over the comparative compositions DROPSTAR®, HYALISTIL®, XILOIAL®, HYABAK®, and VISMED®.

Example 4 - Drop weight reproducibility

A microemulsion composition was prepared according to Example 1 and transferred in a multidose eye drop container. Then, 10 drops of the microemulsion (ME) were erogated to a polymeric PE substrate one after another without wiping of the container tip. Each drop was weight separately using an analytical balance.

As comparative example, water was erogated from a multidose eye drop container and weight accordingly.

The droplet weights are displayed in Table 3.

**TABLE 3**

| Drop | m(ME) in mg | m(water) in mg |
|---|---|---|
| 1 | 24.2 | 27.3 |
| 2 | 25.1 | 28.8 |
| 3 | 24.7 | 25.2 |
| 4 | 27.0 | 25.8 |
| 5 | 26.8 | 29.1 |
| 6 | 26.6 | 29.2 |
| 7 | 26.3 | 28.7 |
| 8 | 26.6 | 29.7 |
| 9 | 27.1 | 28.6 |
| 10 | 27.5 | 30.4 |
| average weight (coefficient of variation CV) | 26.2 $\pm$ 1.1 mg CV 4.3% | 28.3 $\pm$ 1.7 mg CV 5.9% |

To investigate long term stability and reproducibility, drop weights were determined daily for one month as displayed in Table 4.

**TABLE 4**

| Day | m(ME) in mg | Day | m(ME) in mg |
|---|---|---|---|
| 1 | 24.3 | 16 | 25.8 |
| 2 | 22.6 | 17 | 24.9 |
| 3 | 24.6 | 18 | 25.8 |
| 4 | 26.1 | 19 | 21.4 |
| 5 | 26.0 | 20 | 23.7 |
| 6 | 25.9 | 21 | 27.7 |
| 7 | 24.4 | 22 | 24.9 |
| 8 | 26.0 | 23 | 26.6 |
| 9 | 25.0 | 24 | 24.8 |
| 10 | 25.4 | 25 | 28.2 |
| 11 | 21.0 | 26 | 23.9 |
| 12 | 24.7 | 27 | 26.0 |
| 13 | 22.4 | 28 | 22.2 |
| 14 | 24.6 | 29 | 24.3 |
| 15 | 21.6 | 30 | 23.5 |

As can be seen from the drop weights displayed in table 3, drop weight reproducibility of the microemulsion according to the invention is very high, corresponding to that of pure water.

Even after storage of up to 30 days, reproducibility can be maintained, giving an overall average drop weight of 24.6 $\pm$ 1.8 mg (CV: 7.1 %). A high reproducibility is particularly important in ophthalmic compositions in order to provide a constant dosage and treatment of an ophthalmic condition or disease.

Example 5 - Contamination challenge test

A microemulsion composition was prepared according to Example 1 and transferred in a multidose eye drop container. Then, the container tip was contacted with a highly contaminated environment (10x6 CFU/ml of Staphylooccus Aureus) and a drop of microemulsion was erogated to a PBS culture medium. The culture medium was incubated for 7 days at 37 °C and potential contamination was determined by counting the Staphylococcus Aureus CFU/ml concentration.

Moreover, said multidose container was incubated for 7 days at 37 °C and potential contamination was determined by counting the Staphylococcus Aureus CFU/ml concentration of erogated drops.

For both samples, sterility of the examined microemulsion was confirmed.

Example 6 - Cell viability

A microemulsion composition was prepared according to Example 1 and transferred in a multidose eye drop container. Then, a standard MTT assay using human cornea epithelium (HCE) cells was performed, wherein cells were exposed either to a single drop of microemulsion for 24 h (ME, single treatment), or to a single drop twice daily for three consecutive days (ME, repeated treatment for 72 h).

As comparative example, cell viability after treatment with saline solution (negative control, NC), with a 0.01% benzalkonium chloride (BAK) aqueous solution, and with the cationic microemulsion Cationorm® was examined accordingly.

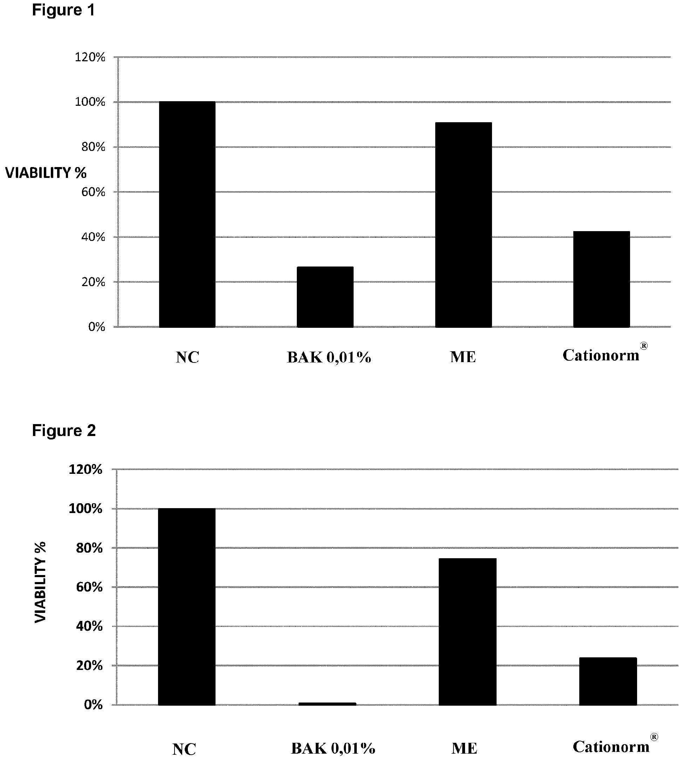

The determined percentage cell viability of human cornea epithelium cells after single and repeated application is displayed in Figures 1 and 2, respectively. The microemulsion according to the invention showed a significantly higher cell viability value, both in single and repeated application compared to the preservative solution (BAK) and the preservative-free commercial product Cationorm®.

Example 7 - *In vivo* tolerability

5 male Cynomolgus macaques were used in a controlled environment (Toxikon laboratories, Toxikon Corp. MA, USA); they received on each eye one drop (30 µl) of the microemulsion each day for 4 consecutive days.

A qualified examiner using a slit lamp performs ocular examination. The results are scored according to a Classification System for Grading of Ocular Lesions combining Draize and McDonald-Shadduck Scoring Systems.

In tables 5-8 the pre-dose data and the data obtained 24 h after the last dose of microemulsion are shown.

TABLE 5: Pre-dose evaluation of the eye

| Animal Number | Eye | Conjunctiva | | | Cornea | | Iris |
|---|---|---|---|---|---|---|---|
| | | Congestion (0-3) | Chemosis (0-4) | Discharge (0-3) | Opacity (0-4) | Area (0-4) | (0-4) |
| 1 | Right | 0 | 0 | 0 | 0 | 0 | 0 |
| | Left | 0 | 0 | 0 | 0 | 0 | 0 |
| 2 | Right | 0 | 0 | 1 | 0 | 0 | 0 |
| | Left | 0 | 0 | 1 | 0 | 0 | 0 |
| 3 | Right | 0 | 0 | 0 | 0 | 0 | 0 |
| | Left | 0 | 0 | 0 | 0 | 0 | 0 |
| 4 | Right | 0 | 0 | 0 | 0 | 0 | 0 |
| | Left | 0 | 0 | 0 | 0 | 0 | 0 |
| 5 | Right | 0 | 0 | 0 | 0 | 0 | 0 |
| | Left | 0 | 0 | 0 | 0 | 0 | 0 |

TABLE 6: Evaluation of the eye 24 hours after the last dose of microemulsion

| Animal Number | Eye | Conjuctiva | | | Cornea | | Iris |
|---|---|---|---|---|---|---|---|
| | | Congestion (0-3) | Chemosis (0-4) | Discharge (0-3) | Opacity (0-4) | Area (0-4) | (0-4) |
| 1 | Right | 0 | 0 | 0 | 0 | 0 | 0 |
| | Left | 0 | 0 | 0 | 0 | 0 | 0 |
| 2 | Right | 0 | 0 | 1 | 0 | 0 | 0 |
| | Left | 0 | 0 | 1 | 0 | 0 | 0 |
| 3 | Right | 0 | 0 | 0 | 0 | 0 | 0 |
| | Left | 0 | 0 | 0 | 0 | 0 | 0 |
| 4 | Right | 0 | 0 | 0 | 0 | 0 | 0 |
| | Left | 0 | 0 | 0 | 0 | 0 | 0 |
| 5 | Right | 0 | 0 | 0 | 0 | 0 | 0 |
| | Left | 0 | 0 | 0 | 0 | 0 | 0 |

TABLE 7: Pre-dose evaluation of the eye

| Animal Number | Eye | Fluorescein Staining (0-4) | Pannus (0-2) | Aqueous Flare (0-3) | Pupillary Light Reflex (0-2) | Lens (0-1) |
|---|---|---|---|---|---|---|
| 1 | Right | 0 | 0 | 0 | 0 | 0 |
| | Left | 0 | 0 | 0 | 0 | 0 |
| 2 | Right | 0 | 0 | 0 | 0 | 0 |
| | Left | 0 | 0 | 0 | 0 | 0 |
| 3 | Right | 0 | 0 | 0 | 0 | 0 |
| | Left | 0 | 0 | 0 | 0 | 0 |
| 4 | Right | 0 | 0 | 0 | 0 | 0 |
| | Left | 0 | 0 | 0 | 0 | 0 |
| 5 | Right | 0 | 0 | 0 | 0 | 0 |
| | Left | 0 | 0 | 0 | 0 | 0 |

TABLE 8: Evaluation of the eye 24 hours after the last administration of microemulsion

| Animal Number | Eye | Fluorescein Staining (0-4) | Pannus (0-2) | Aqueous Flare (0-3) | Pupillary Light Reflex (0-2) | Lens (0-1) |
|---|---|---|---|---|---|---|
| 1 | Right | 0 | 0 | 0 | 0 | 0 |
| | Left | 0 | 0 | 0 | 0 | 0 |
| 2 | Right | 0 | 0 | 0 | 0 | 0 |
| | Left | 0 | 0 | 0 | 0 | 0 |
| 3 | Right | 0 | 0 | 0 | 0 | 0 |
| | Left | 0 | 0 | 0 | 0 | 0 |
| 4 | Right | 0 | 0 | 0 | 0 | 0 |
| | Left | 0 | 0 | 0 | 0 | 0 |

(continued)

| Animal Number | Eye | Fluorescein Staining (0-4) | Pannus (0-2) | Aqueous Flare (0-3) | Pupillary Light Reflex (0-2) | Lens (0-1) |
|---|---|---|---|---|---|---|
| | Right | 0 | 0 | 0 | 0 | 0 |
| 5 | Left | 0 | 0 | 0 | 0 | 0 |

No marked changes were found during clinical observation and ocular examination of different eye parts, i.e. conjunctiva (congestion, chemosis, discharge), cornea (opacity, area), iris, lens, aqueous flare, pannus, in the eyes of the treated animals at all times of observation after treatment with the microemulsion according to the invention.

Example 8 - *In vitro* model of dry eye syndrome

1. Introduction

An *in vitro* model of dry eye syndrome that re-creates the impairment of water flux within the epithelial water channels during dryness was used to investigate the efficacy of the commercially available ophthalmic compositions Cationorm® (P1), Artelac® Splash (P2), and Systane® UD (P3), as well as the microemulsion according to Example 1 (P4) in counteracting dehydration via water channel hydration and in homeostasis recovery.

The commercially available reconstructed human corneal epithelium (HCE) of 0.5 cm$^2$ from SkinEthic was used to set up an *in vitro* DES model by modifying the culture conditions in order to induce the morphological, cellular and biochemical modifications related to the dry eye symptoms: inflammation, modification of the structural compartments, expression of relevant markers and microvilli "network", thus mimicking a reversible and not severe corneal dryness.

Two different protocols were applied:

- the compositions P1-P4 were applied on a series of HCE tissues and immediately transferred in controlled conditions (low RH < 40 %, T = 40 °C) for 36 h in order to induce dryness stress (pre-treatment series).
- the compositions P1-P4 were applied on a series of DES models issued from 36 h of dryness stress and further cultivated in standard conditions for 6 h and 24 h (post-treatment series).

The following parameters were assessed on both series:

- barrier function permeability
- gene expression of specific biomarkers
- histo-morphological and scanning electron microscopy (SEM) analysis

and compared with HCE tissues treated with saline solution and cultured in standard conditions (negative control) and DES models cultured in dryness conditions for 36 h and recovered in standard culture conditions for 6 and 24 h (positive control).

2. Experimental design

After 2-3 hours in standard culture conditions to allow the HCE tissues to recover from storage, two different protocols have been performed to address:

- the preventive efficacy of the compositions (Pre-treatment series)
- composition efficacy in restoring homeostasis (Post-treatment series)

PRE-TREATMENT SERIES

After tissue recovery, 15 µL of the respective composition was applied on the surface of a HCE tissue and the tissue was immediately transferred in the defined controlled conditions (low relative humidity (RH) < 40 %, T = 40°C) for 36 h in order to induce dryness stress. This protocol was used to assess the efficacy of the product in counteracting dryness.

POST-TREATMENT SERIES

After tissue recovery, HCE tissues were transferred in the defined controlled conditions (low RH < 40 %, T = 40°C), used to induce dryness stress for 36 h. After 36 h in dryness conditions, 15 $\mu$L of the respective composition was directly and uniformly applied on the DES model and the model was further cultivated in standard culture conditions for 6 h and 24 h to assess product efficacy in restoring homeostasis.

The results were compared to:

- Negative control (NC): HCE tissue treated with saline solution (0.9% NaCl, 15 $\mu$L) and cultured in standard conditions for 36 h and recovered for 6 h and 24 h always in standard culture conditions.
- Positive control (PC; dryness): HCE tissue cultured in dryness conditions ($CO_2$ incubator with RH < 40 %, T = 40°C) for 36 h and recovered in standard culture conditions (37 °C, 5% $CO_2$) for 6 and 24 h.

Before and after treatment, for both series, the barrier function properties were assessed by transepithelial electrical resistance (TEER) measurement (only on controls at t = 0 h for both series, and at t = 36 h for the two post-treatment series).

At the end of the exposure period, for both series, the DES models were rinsed with saline and the samples were prepared for the following analysis:

- Gene expression by qRT-PCR of a specific gene-signature including: biomarkers of inflammation (TNF-$\alpha$), water channels (AQP-3), extracellular matrix modelling (MMP-9) and defense mucin (MUC-4) (in duplicate)
- Histo-morphology by hematoxylin and eosin (H&E) staining (in simplicate) while the correspondent media were collected and stored at -20°C for further analysis.

3. Test system

The EPISKIN Reconstituted Human Corneal epithelium of 0.5 cm$^2$ (HCE tissue) was used for the evaluation. Briefly, epithelial human immortalized cells (HICEC) were deposed on a polycarbonate filter and cultured at the air-liquid interface for 5 days in a chemically defined medium in standard conditions in order to form a structured epithelium.

The model was validated by EURL ECVAM (SkinEthic™ HCE Eye Irritation Test Method) as alternative method to identify chemicals not requiring classification and labelling for eye irritation or serious eye damage as displayed in Table 9 below.

TABLE 9: Evaluation of DES models

| NAME | Reconstituted Human Corneal Epithelium HCE/S/5 | STATE |
|---|---|---|
| BATCH | 17-HCE-042 | |
| MANUFACTURER | EPISKIN | |
| THICKNESS AT DAY 5 | 75µm | |
| HISTOLOGY AT DAY 5 (Number of cell layers ≥ ) | 6 cell layers | Accepted |
| CELL VIABILITY AT DAY 5 (1≤ O.D≤ 2.5) | O.D. = 2.0 (CV= 5.9 %) | Accepted |
| IC50 DETERMINATION (1mg/mL ≤ IC50 ≤ 3.5mg/mL) | 2mg/mL | Accepted |

Then, the HCE tissues were removed from the agarose nutrient solution under a sterile air flow cabin and rapidly transferred to a 6-well plate previously filled with maintenance medium (1 mL/well) at room temperature and incubated at 37°C, 5% $CO_2$, saturated humidity overnight.

4. Methods

4.1 Histo-morphological analysis

The histological evaluation is a complementary endpoint, useful to confirm the physical and molecular investigations and for a deeper understanding of the interaction between the investigated composition and the living tissue.
At the end of the treatment, HCE tissues were rinsed with saline solution and fixed in 10% formalin. Samples were included in paraffin blocks and sections of 5 $\mu$m were obtained. Slides were stained with standard H&E following VitroScreen procedure.
The histological samples were analyzed under light microscopy (40x): the overall morphology and its modification compared to the negative controls were analyzed on at least three sections of the same tissue.

4.2 TEER measurement

TEER (trans-epithelial-electrical-resistance) is an indirect assessment of tight junction stability and consequently is a direct measure of the functionality of barrier function in epithelial tissue: it reflects the global resistance of the barrier linked to the structure and the tissue thickness.
After the above describe treatment procedure and removal of the composition to be analyzed, trans-epithelial-electrical resistance was measured according to standard protocols.
The Raw Data of $\Omega$ are recorded and further processed. The Biological replicate mean was calculated on three technical replicates and corrected considering the tissue surface (0,5 cm$^2$) according the following formula:

$$\Omega \text{ (mean 3 measurements) sample x tissue surface (0.5 cm}^2)$$

The biological replicate $\Omega$ mean was then calculated. The test item results were compared to the negative control.

4.3 Real time PCR

Real time PCR was performed using Applied Biosystems 7500 Fast Real Time PCR with fluorescent-based PCR: Taqman assays.
The RNAqueous method is a rapid, phenol free, filter based RNA isolation system used to extract the total RNA form cellular samples. The High Capacity cDNA Reverse Transcription kit was used to synthetize cDNA from RNA. Relative quantification determines the change in the expression of a nucleic acid sequence in a test sample relative to the same sequence in a calibrator sample. GAPDH was used as an endogenous control gene to normalize input amounts. RNA integrity was evaluated by loading the extracted RNA on agarose gel 1%: ribosomal band 18S and 28s were detected.

Procedure

At the end of both treatments (36 h + 6 h and 36 h + 24 h), tissues were collected in lysis buffer for RNA extraction, cDNA retrotranscription and gene expression analysis according to standard protocols.
Fluorescence data of the RT-PCR generated by the thermocycler ABI 7500 Fast were collected by the internal software SDS 2.0.6. Because each cycle in the PCR reaction corresponds to a 2-fold increase in PCR product, a difference of one in threshold cycle number represents a 2-fold difference in the expression of a particular gene compared to the calibrator sample and can be considered as significant. 95% of confidence level is used by the software to calculate the errors. A value has been accepted as significant when the gene is "one fold" up (relative quantification (RQ) > 2) or down regulated (RQ < 0.5) compared to the calibrator sample (RQ = 1). The internal instrument level of confidence used was 95%.

5. Results

5.1 Histological analysis: H&E staining

Three vertical tissue sections were prepared on each histological slide. On one selected slide three microscopic acquisitions (40x) of three selected parts were performed. For each sample the most representative acquisition of the selected vertical section is reported herein.
The morphology of control tissues (NC) was not significantly modified during the culture time and as expected, the HCE tissue thickness was increased during this time confirming a good proliferative status.

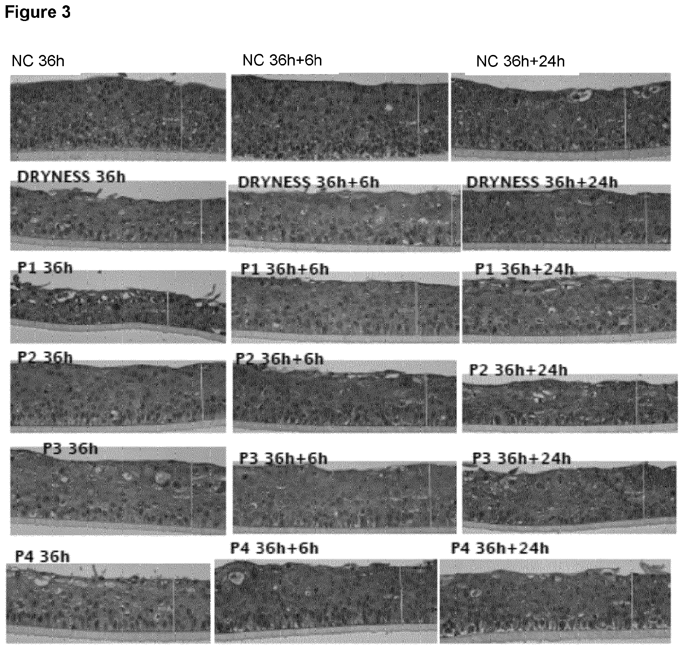

As displayed in Figure 3, the dryness control (positive control) immediately at the end of the stress induction period presented the following morphological modifications compared to the negative control:

- Significant reduction of HCE tissue thickness
- Squamous layer structure impairment
- Dense and compact extracellular matrix
- Few picnotic nuclei and modified cell to cell connections

In the post dryness stress period the recovery of the reference morphology was partially observed: in particular after 6 h the HCE tissue thickness was slightly increased but the extracellular matrix showed signs of toxicity; after 24 h the superficial squamous layer presented an abnormal production of mucines (dark purple color) as defense mechanism to recover tissue integrity.

Pre-treatment series

The DES model treated with P1 showed reduced HCE tissue thickness, damages and signs of toxicity at the squamous layer.
The DES model treated with P2 showed a morphology and thickness not different from the negative control.
The DES model treated with P3 showed a morphology and thickness not different from the negative control, but significant signs of cell swelling and extracellular matrix modifications were visible at the wing cells layer.
The DES model treated with P4 showed a morphology and thickness not different from the negative control, thus providing good protection against dryness stress.

Post-treatment series

The effect of treatment on dry HCE tissue during the recovery periods of 6 h and 24 h dependent on the applied composition (P1, P2, P3, and P4) is displayed in Figure 3.
The DES model treated with P1 showed recovery of a reference morphology early after 6h but in presence of reduced HCE tissue thickness and significant signs of toxicity at the squamous layer.
The DES model treated with P2 showed recovery of a reference morphology early after 6 h but with reduced HCE thickness and significant signs of toxicity at the extracellular matrix and squamous layer, which was more evident in the 24 h sample.
Morphology and thickness according to the negative control were partially restored in the DES model treated with P3; however in the 24 h read out, the extracellular matrix appeared modified and the production of defense mucines increased.
Morphology and thickness of the negative control were early (after 6 h) restored in the DES model treated with P4, indicating that the product determined a good recovery of the damages induced by dryness stress; in the 24h read out, the extracellular matrix appears only slightly modified.

5.2 Real time PCR

Biological role of the detected biomarkers:

**TNF-$\alpha$** overexpression is crucial in tissue inflammatory response. TNF alpha is a cytokine expressed constitutively or as a result of stimulation. TNF alpha gene transcription is premature and immediate following stimulation. This polypeptide is a primary mediator of inflammation. Depending on its concentration, duration of cell exposure and the presence of other mediators, the complex network of its biological effects can determine both, local and systemic beneficial effects or damages.
**MMP-9** is the most important gelatinase present on the ocular surface. This enzyme lyses a variety of different substrates including components of the corneal epithelial basement membrane and tight junction proteins (such as ZO-1 and occludin) that maintain corneal epithelial barrier function. It appears to play a physiological role in regulating corneal epithelial desquamation. High levels of MMP-9 are dosed in tear fluids of patients with keratoconjunctivitis sicca (KCS), in particular in patients with ulceration. Tear MMP-9 activity levels directly correlated with the severity of corneal disease. The increased MMP-9 activity in KCS may be responsible in part for the impaired corneal epithelial barrier function, increased corneal epithelial desquamation, and corneal surface irregularity.
**MUC-4** is a mucin that plays a role in maintenance of water on the surface of the eye. Mucins network keeps the ocular surface wet and protected from adverse environmental conditions. Their function at the ocular surface has been ascribed to secreted gel-forming mucins acting as lubricating agents and clearing molecules. There is some evidence that DES leads to an alteration in ocular mucins. MUC-4 upregulation can be interpreted as an early marker that acts as a defense signal to stimulate the production of mucins.

**AQP-3** is an integral membrane protein that transports water across cell membranes in response to osmotic gradients. AQP-3 is expressed in the corneal epithelium at lower levels and primarily in the proliferating basal cell layer. At the ocular surface, AQP-3 upregulation is predicted to accelerate epithelial resurfacing. The increase of AQP-3 is a positive effect to stimulate the increase of water content with the result of tear osmolarity stabilization. In dryness conditions AQP-3 is overexpressed and dislocated from the basal layer to the upper layers of the tissue reflecting high membrane water permeability and confirming AQP-3 functional role in modified water channels.

All data were normalized to the negative control (set as relative quantification (RQ) = 1). $RQ \leq 0.5$ was classified as significant down-regulation, whereas a $RQ \geq 2$ was classified as significant up-regulation. A composition was defined to have positive efficacy against dryness stress or in recovering dryness stress compared to the dryness (positive) control, if TNF-$\alpha$ was down/not regulated, AQP-3 was not regulated, MMP-9 was down/not regulated and MUC-4 was not regulated.

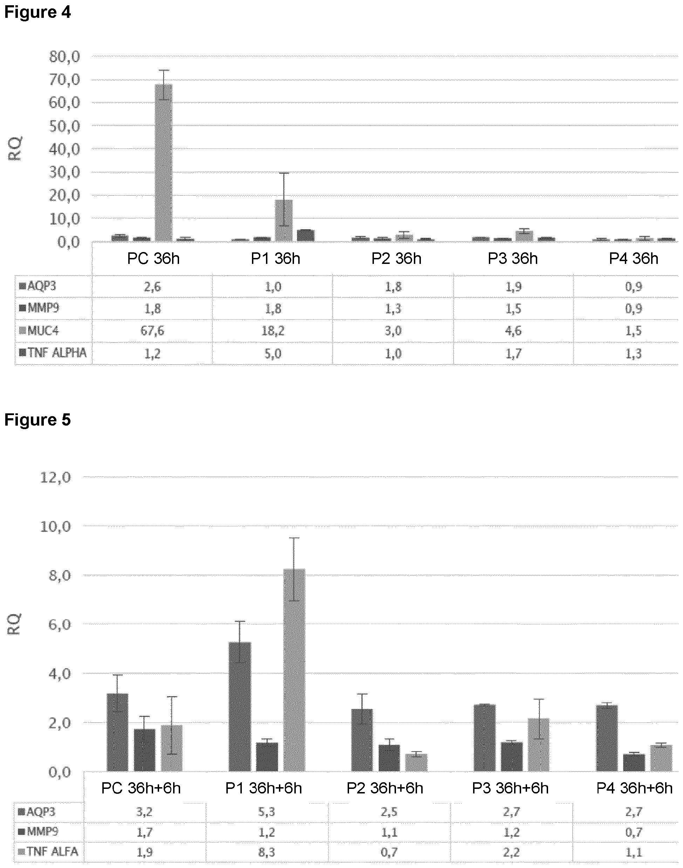

In Fig. 4 the RQ results for the pre-treatment series are reported.

The positive control (PC) immediately after the end of the stress showed significant up-regulation of AQP-3, MMP-9, and MUC-4. The results reflect a damage in the water retention capacity and defense against ocular surface barrier damage. The TNF-$\alpha$ was not expressed at this time.

A positive efficacy in maintaining a homeoastic behavior during the induction of the dryness stress can be attributed to the microemulsion according to the invention (P4), where no regulation of the target genes was detected.

P1 induced an over expression of TNF-$\alpha$ related to product toxicity (as underlined by histology) and a reduced expression of MUC-4 compared to the dryness (positive) control.

P2 and P3 globally showed the same features, namely a reduced expression of MUC-4, and no effects on AQP-3, MMP-9 and TNF-$\alpha$. However, P3 with a RQ value of 1.7 for TNF-$\alpha$ was close to a significant upregulation level.

In Fig. 5 the RQ results for the post-treatment series 36 h + 6 h are reported.

AQP-3 was still upregulated in the positive control (PC) 6 h after stress, indicating an imbalance in water retention capacity. MMP-9 and TNF-$\alpha$ transcriptional activity were close to the significancy level suggesting an impaired HCE homeoastasis.

P1 was not effective in reducing the inflammatory pathway and the AQP-3 imbalance, showing no effect in counteracting the dryness stress based on the genes over expression.

P2 and P3 both slightly reduced the AQP-3 and MMP-9 over-expression compared to the positive control. Further, P3 was associated with a significant over expression of TNF-$\alpha$.

The microemulsion according to the invention (P4) was effective in reducing the AQP-3 expression level and significantly effective in restoring the HCE homeoastatic balance (no regulation of MMP-9 and TNF$\alpha$).

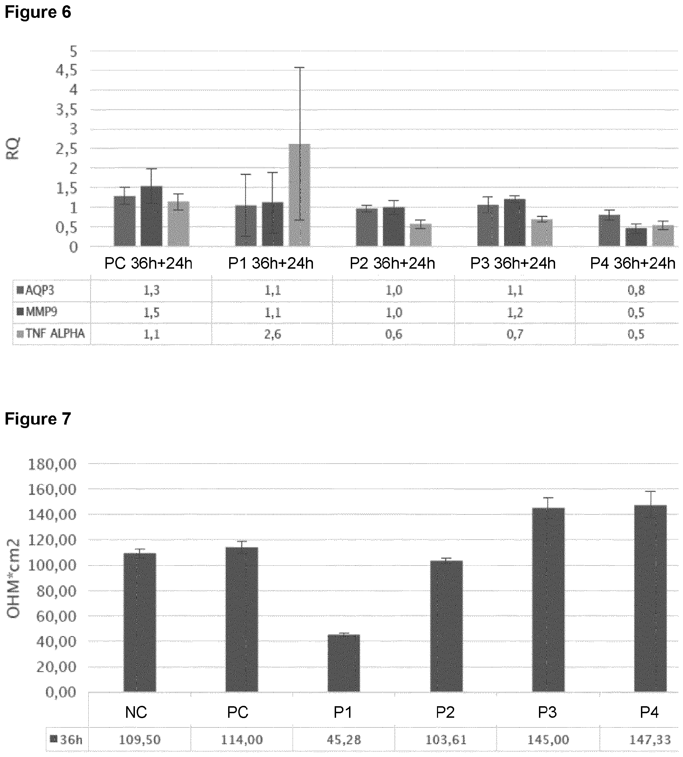

In Fig. 6 the RQ results for the post-treatment series 36 h + 24 h are reported.

The positive control (PC) has recovered a physiological expression of the target genes (no significant expression levels) indicating a recovery from the dryness stress.

P1 was still determining an inflammatory stimulation.

P2 and P3 showed an efficacy not different from the positive control.

The microemulsion according to the invention (P4) induced a down-regulation of MMP-9 and TNF-$\alpha$ demonstrating a high anti-inflammatory efficacy.

5.3 TEER measurement: Barrier function properties

The trans-epithelial electrical resistance (TEER), a physical parameter of the HCE barrier integrity, was used to characterize the dryness model and the compositions' influence on ion paracellular flux at mucosal epithelium level during (pre-treatment series) and after (post-treatment series) stress induction.

The TEER values before treatment (t = 0 h) reflect the global resistance of the barrier linked both, to the integrity of tight junction structure and to the epithelial thickness.

In Figure 7 are reported the results of TEER expressed in Ohm*cm$^2$ after 36 h in dry conditions for the pre-treatment series.

No significant modification was observed in the TEER values of the DES model incubated in dry conditions (PC) compared to the negative control (NC).

P1 showed a significant TEER reduction compared to the dry control (- 60%).

P2 showed no significant modification compared to the dry control (- 9%).

P3 and the microemulsion according to the invention (P4) showed a significant TEER increase compared to dry control tissues (+ 27% and + 29%, respectively).

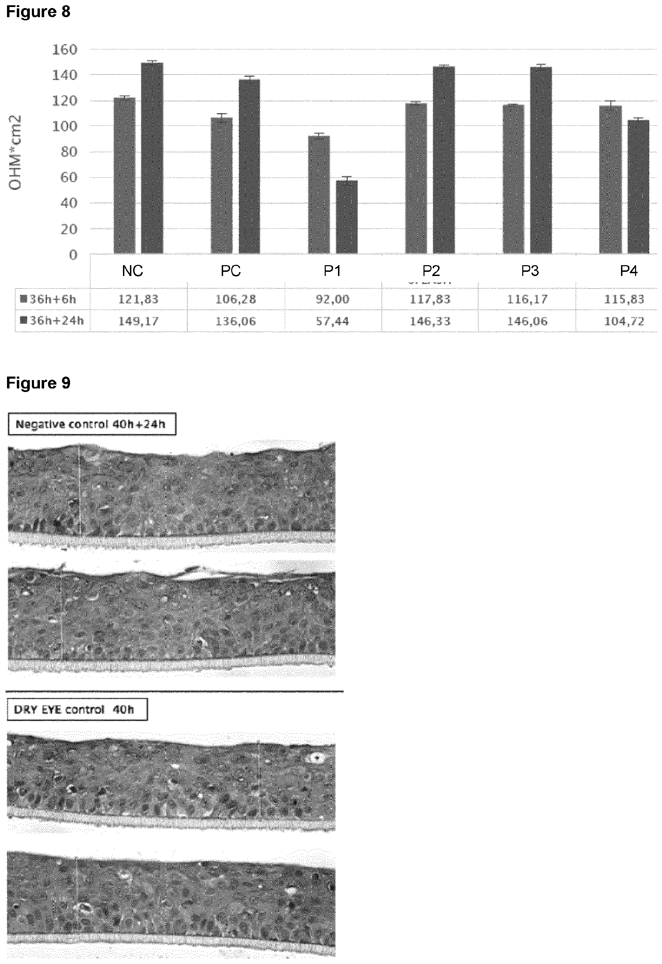

In Figure 8 are reported the results of TEER expressed in Ohm*cm$^2$ after 36 h dryness + 6 h and 24 h treatment in standard culture conditions for the post-treatment series.

No significant modification was observed in the TEER values of the HCE tissue incubated in dry conditions (PC) compared to the negative control (NC).

After 36 h in dry condition + 6 h treatment in standard condition no significant modification was observed compared to the dry control with all compositions tested.

After 36 h in dry condition + 24 h treatment in standard condition with

- P1 a significant TEER reduction was observed compared to the dry control (-58%);
- P2 and P3 no significant modification was observed compared to the dry control (+ 7% and + 8%, respectively);
- P4 a TEER reduction was observed compared to the dry control (- 23%).

Example 9 - *In vitro* model of dry eye syndrome

1. Introduction

In Example 9, the above describes HCE dry eye model was used to evaluate and compare the efficacy of an inventive microemulsion prepared according to Example 1 (P1) and the commercially available ophthalmic composition XIIDRA® Lifitegrast 5% (reference product, P2).

The compositions P1 and P2 were applied on HCE tissues, which were previously issued to dry stress for 40 h, and further cultivated in standard conditions for 24 h.

The following parameters were assessed:

- gene expression of the biomarkers:

  - AQP-3, which plays a role in water channels regulation;
  - MMP-9, which plays a role in extra cellular matrix degradation;
  - ICAM-1, which plays a role in the inflammatory response;
  - TNF-$\alpha$, which plays a role in the inflammatory response;
  - TLR4, which plays a role in the inflammatory response;
  - CD44, which plays a role in the regulation of hyaluronic acid uptake

- histological analysis (H&E staining)

The results of the DES models treated with the compositions P1 and P2 were compared with HCE tissues cultured in standard conditions and treated with saline solution (negative control) and HCE tissues cultured in dryness conditions for 40 h, and recoverd and treated with saline solution for 24 h in standard culture conditions (positive DES control).

2. Experimental design

HCE tissues were prepared as described above (cf. Example 8) and transferred in defined controlled conditions (low RH < 40 %, T = 40 °C), used to induce dryness stress, for 40 h.

Then, 15 $\mu$L of composition P1 or P2 was directly and uniformly applied on a DES model and the DES model was further cultivated in standard culture conditions for 24 h to assess product efficacy in restoring homeostasis.

The results were compared to:

- Negative control (NC), i.e. HCE tissue cultured in standard conditions for 40 h, then treated with saline solution (0.9 % NaCl, 15 $\mu$L), and recovered in standard culture conditions for 24 h.
- Positive control (DES): HCE tissue cultured in dryness conditions ($CO_2$ incubator with RH < 40 %, T = 40 °C) for 40 h, then treated with saline solution (0.9% NaCl, 15 $\mu$L) and recovered in standard culture conditions for 24h.

At the end of the exposure period, the DES models were rinsed with saline solution and samples were prepared for the following analyses:

- Gene expression by RT-qPCR of a specific gene signature influenceing water channels (AQP-3), extracellular matrix modelling (MMP-9), inflammation (TNF-$\alpha$, ICAM-1, TLR-4) and regulation of hyaluronic acid uptake (CD44) (in triplicate)
- Histo-morphology by H&E staining (in duplicate)

Histo-morphological analysis and real time PCR was performed as described in Example 8.

3. Results

3.1 Histological analysis: H&E staining

Three vertical tissue sections were prepared on each histological slide; on one selected slide three microscopic acquisitions of three selected parts were performed for each biological replicate. For each sample representative acquisitions of the selected vertical section are reported herein.

The morphology of negative control models was not significantly modified during the culture time and, as expected, the HCE tissue thickness was increased during this time confirming a good proliferative status (cf. Figure 9).

The dryness control (positive control) presented the following morphological modifications compared to the negative control:

- reduction of HCE tissue thickness
- few picnotic wing cells
- reduced mucin production by superficial cell layer

In the post dryness stress period the recovery of the reference morphology was partially observed. The superficial layer presented mucin formation around the cells and also the HCE tissue thickness increased to values similar to the negative control after 40 h + 24 h treatment.

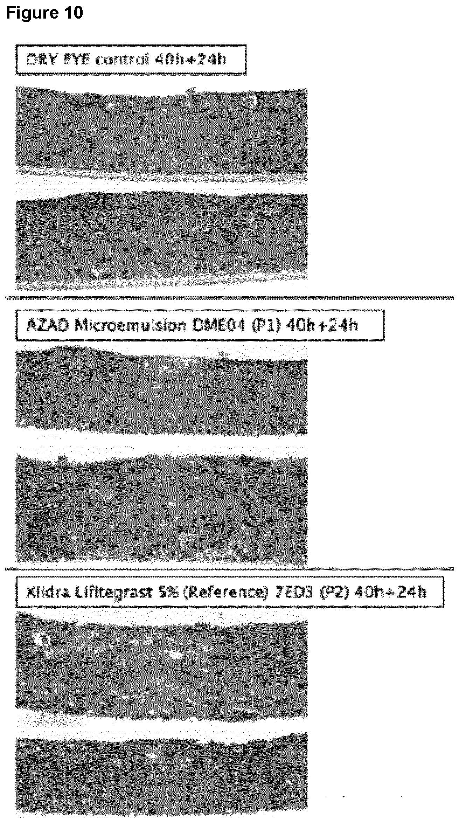

The effect of treatment on dry HCE tissue during the recovery period of 24 h dependent on the applied composition (P1 and comparative composition P2) is displayed in Figure 10.

The DES model treated with microemulsion P1 according to the invention showed perfectly conserved basal cells, matrix remodeling with non-modified wing cells, and a slightly increased tissue thickness compared to the negative control after 40 h + 24 h treatment.

The DES model treated with P2 showed partially conserved basal cells with few picnotic nuclei, wing cell swelling with few picnotic nuclei, a significant modification of epithelial integrity of the superficial cell layer, and a slightly increased tissue thickness compared to the negative control after 40 h + 24 h treatment.

3.2 REAL TIME PCR

The biological role of the selected biomarkers is described in Example 8 (MMP-9, AQP-3, TNF-$\alpha$) and below.

**CD44** (Hyaluronic acid receptor) is an ubiquitously expressed cell surface proteoglycan and the major cell surface receptor for hyaluronic acid (HA). HA is a high-molecular-weight linear polymer and is the principal component of the extracellular matrix. It serves a variety of functions, including space filling, lubrication of joints and provision of a matrix through which cells can migrate, providing continuous moisture by binding up to 1000 times its weight in water. HA also functions as a manipulator in the process of epithelial proliferation, which is essential in normal epidermal function, as well as during reepithelization in tissue repair.

**TLR4** (Toll-like receptor 4) is a pattern recognition receptor that stimulates the activation of NF-kB, which upregulates the production of proinflammatory cytokines with induction of innate and adaptive immune responses. In DES, the degradation of extracellular matrix components including fibronectin, hyaluronic acid, and heparan sulfate generates endogenous bioactivators of TLR4. As a consequence, TLR4 expression is up-regulated in DES, with increase of the inflammatory response to desiccating stress at the ocular surface.

**ICAM-1** (Intercellular adhesion molecule 1, also known as CD54) is considered a signaling molecule, favoring the ocular surface inflammation by the recruitment potential antigen presentation by epithelial cells. Increased endogenous ICAM-1 expression is detected in the epithelial cells present in the conjunctival and accessory lacrimal tissues in DES patients. ICAM-1 upregulation is triggered by IL-1$\alpha$ and TNF-$\alpha$, and stimulates the recruitment of leukocytes to the inflamed ocular site through the recognition of the receptor LFA1, on the leukocytes surface.

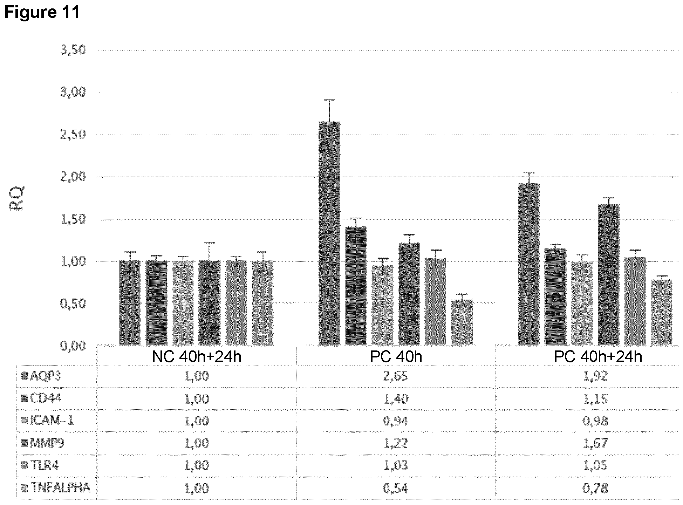

Fig. 11 displays the relative quantification (RQ) values of the control tissues subjected to dryness stress (PC, 40 h), and post-incubated in standard conditions for 24 h. The results are expressed with respect to the negative control (NC 40 h + 24 h, set as RQ = 1, see Example 8) and reflect the establishment of the DES model after 40 h of dryness stress and the following recovery phase.

Immediately after the 40 h stress induction, the DES positive control showed a significant up-regulation of AQP-3. AQP-3 over-expression reflects a dysregulation in the water retention capacity and increased membrane water permeability by modification of the water channels, confirming that the DES model has been established. This finding was also evidenced by a reduced thickness of the HCE tissue caused by the dryness stress, visible at histological level.

After a recovery period of 24 h, AQP-3 was still upregulated in the DES positive control (however, at non-significant expression levels), indicating a recovery from the dryness stress. The other genes under investigation were not significantly regulated, however MMP-9 expression appears slightly increased compared to DES at 40 h.

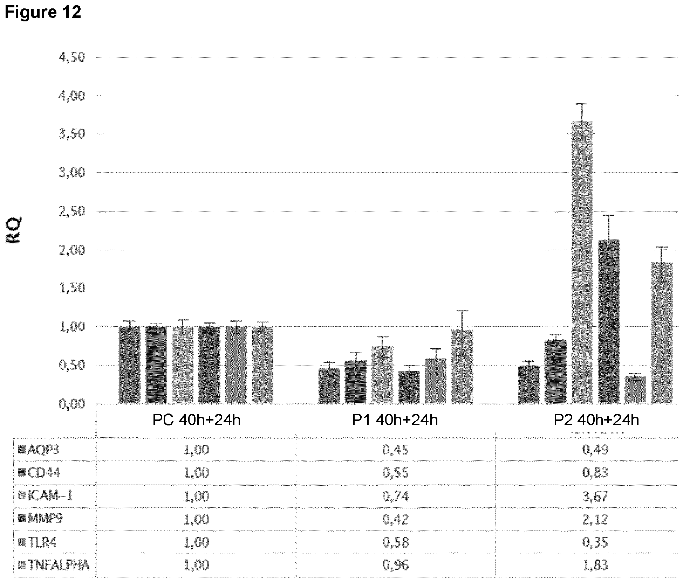

In Fig. 12 the RQ values of the tissues subjected to dryness stress (PC) and treated with the test compositions P1 and P2 for 24 h during the post-incubation period in standard conditions are shown. The results are expressed with respect to the DES control (PC 40 h + 24 h was set as 1).

The treatment with P1 and P2 was effective in reducing the water permeability imbalance, displayed by down-regulation of AQP-3.

MMP-9 downregulation was observed after the treatment with P1. This decrease indicates a positive effect of the microemulsion according to the invention in inhibiting the matrix and tight junction protein degradation leading to impairment of the corneal barrier function and desquamation.

The opposite result was observed after the treatment with comparative composition P2, where the up-regulation of MMP-9 indicated that P2 may cause an irregular extracellular matrix and tight junction protein degradation, favoring a delay in the corneal damage healing.

The upregulation of ICAM-1, observed after the treatment with P2, suggests that this composition could favor ocular surface inflammation, facilitating the potential recruitment of leukocytes to the inflamed ocular surface. This result needs however to be contextualized in this *in vitro* model: the mechanism of action of Xiidra® is in fact based on the binding of LFA, the ICAM-1 receptor on the leukocytes membrane, with inhibition of the inflammatory response (i.e. recruitment of leukocytes in the site of inflammation). Since this model does not include the immunological component (immune cells such as leukocytes), the up-regulation of ICAM-1 should not influence the downstream inflammatory response. Moreover, ICAM-1 up-regulation is not correlated with TLR4 activation, which was found downregulated indeed, indicating that Xiidra® Lifitegrast has a direct effect as anti-inflammatory agent.

Treatment with P1 did not modulate the genes involved in the inflammatory response and moreover TLR4 was downregulated (with RQ values very close to significancy level) suggesting a global anti-inflammatory effect.

Both products did not show a significant regulatory effect on the hyaluronic acid receptor CD44.

4. Conclusion

The results obtained for both formulations are summarized in the table 9:

TABLE 9

| | P1 | P2 (comparative example) |
|---|---|---|
| **Morphology** (H&E) | The tissue appeared viable and with a preserved morphology → protective effect of the formulation | Modifications of the corneal epithelium surface and few signs of toxicity at basal level |
| **Water balance** (AQP3) | Down-regulation of AQP3 → efficacy in reducing the water permeability imbalance and in restoring the HCE hydration homeostasis | |
| **Extra cellular matrix degradation** (MMP9) | Downregulation of MMP-9 → inhibition of extracellular matrix degradation that has a further protective efficacy against corneal barrier function impairment | Up-regulation of MMP9 → potential irregular extracellular matrix degradation, with delay in the corneal damage healing |
| **Inflammatory response** (ICAM1, TNFα, TLR4) | The genes linked to inflammatory pathways were not regulated or slightly down-regulated in case of TLR4 → anti-inflammatory response | *Upregulation of ICAM-1 without activation of TLR-4 → potential increase of ocular surface inflammation, with recruitment of leukocytes* (this result needs however to be contestualized, cf. supra) |
| **Regulation of HA uptake** (CD44) | No significant regulatory effect on the hyaluronic acid receptor CD44 | |

Example 10 - Evaluation of microemulsion effect on dry eye disease in mice

1. Induction of dry-eye disease (SiccaSystem™)

Dry-eye disease was induced in mice using a combination of desiccating environment and scopolamine administration (SiccaSystem™). Mice were administered scopolamine by placing a small piece of scopolamine patch (approx. 1/12th of a patch) into both ears. Patches were checked twice daily and repositioned, if needed. Every three days, patches were replaced. Concomitantly, mice were placed in a controlled desiccating environment (SiccaSystem™, 5-10% humidity, 15 L/min airflow).

2. Treatment protocol

Treatment was started after 16 days exposure to the Siccasystem™ to induce chronic dry eye disease. The microemulsion and the reference compound Restasis were administered twice daily by topical application to both eyes of one drop (10 $\mu$l) for a period of 12 days.

3. Quantification of corneal epithelial damage

Mice were anesthetized using a cocktail of medetomidine : ketamine : saline of 1 : 1.5 : 2.5, equivalent to 0.6 mg kg$^{-1}$ medetomidine and 45 mg kg$^{-1}$ ketamine, injected subcutaneously at 30 $\mu$l per 10 g body weight. Anesthesia reversal (atipamezole hydrochloride : saline of 1:9) was used on study day 15. A one sixth of one sodium fluorescein strip (equivalent to 166 ng) was placed on the cornea for 10 seconds. Corneal epithelial damage was registered on study day 15 and study day 28 by taking a photograph using a Leica DM MC165FC long working-distance microscope (Leica Microsystems).

Severity of corneal surface inflammation was assessed by blindly scoring fluorescein puncta and patches as

absent = 0;
slightly punctate staining = 1;
strong punctate staining but not diffuse = 2;
small positive plaque areas = 3; and
large area fluorescein plaque = 4.

4. Results

As displayed in Figure 13, ME5% showed a statistically significant improvement of corneal fluorescein staining from day 15 to day 28 (Wilcoxon matched-pairs signed rank test, P < 0.05; Fig. 13B). This reduction was similar to the effect by the reference compound, Restasis® (P < 0.05; Fig. 13D). ME7% showed a statistical trend toward improved corneal fluorescein staining scores (P = 0.17; Fig. 13C). In contrast, corneal fluorescein staining in untreated eyes was not significantly different between days 15 and 28 (P > 0.99; Fig. 13A).

Conclusion

The microemulsion according to the invention was found to exert a very strong activity against the dry eye disease. This healing effect resulted to be multifactorial, i.e. increase of cornea hydration and homeostasis, decrease of water loss and maintenance of tissue interconnecting scaffold, and unexpected decrease of inflammation was observed.

A gene-expression analysis carried out on an *in vitro* human cornea epithelium subjected to very strong DES conditions and subsequently treated with the microemulsion of the invention showed unexpected decrease of the biomarkers related to DES: MMP-9 (enzyme inducing degradation of biopolymeric components of corneal epithelium basement membrane and intercellular tight junctions, leading to cornea desquamation and ulcers), AQP-3 (a carrier protein transporting water molecules, leading to loss of water), CD44 (biochemical controlling concentration of hyaluronic acid, main factor of hydration of tissue and filling of intercellular space), and TLR4 (a cellular membrane toll-like receptor activating production of inflammatory cytokines); this multifactor decrease was much higher than the effect induced by Xiidra® Lifitegrast, a drug recently launched specifically to treat DES (see Figure 12).

In another study on *in vitro* human cornea epithelium with DES induced conditions the treatment with the microemulsion of the invention was compared with CATIONORM® (cationic microemulsion), ARTELAC® SPLASH (hyaluronic acid solution) and SYSTANE® UD (gelling polymer solution). The biomarker MMP-9 resulted to be downregulated by the microemulsion of invention twice more than all the 3 marketed products; the polypeptidic cytokine TNF-alfa, an inflammation-related agent overexpressed by DES, was down-regulated by the microemulsion of the invention by a percentage comparable with the effect of ARTELAC® SPLASH, but twice more than by SYSTAN®E UD and 800% more than by CATIONORM®, which actually resulted to induce an increase of inflammation (see Figure 6)

The very good ophthalmic tolerability of the microemulsion of the invention was proved both *in vitro* (on human cornea epithelium cells) and *in vivo* (on rabbits and monkeys). A comparison with the anti-DES marketed product CATIONORM® (cationic microemulsion) evidentiated very much higher tolerability for the microemulsion of the invention, which resulted to be non-toxic, whereas the cationic microemulsion was found to be toxic for human cornea cells (see Figure 1).

Also other biopharmaceutical parameters of the microemulsion of the invention were found to be improved over other marketed anti-DES products. The wettability, i.e. the spreadability of the microemulsion drops over a polymeric surface simulating the cornea was very much higher than the wettability of XILOIAL®, HYALISTIL®, VISMED®, HYABAK®, and DROPSTAR®. This better potential coverage of the whole cornea surface can lead to stronger efficacy against the different aspects of DES (see Table 2)

The subject-matter of the following items is also comprised by the present invention.

1. Microemulsion comprising

    i. at least one oily component,
    ii. an aqueous phase,
    iii. at least one non-ionic surfactant,
    iv. at least one polysaccharide, a salt or a derivative thereof, and
    v. at least one cross-linking agent.

2. Microemulsion according to item 1, wherein the oily component (i) is present in an amount of 1.0 - 10.0 wt.-% based on the total weight of the microemulsion.

3. Microemulsion according to any one of items 1-2, wherein the oily component (i) is selected from the group consisting of fatty acids and fatty acid esters, particularly unsaturated fatty acids and fatty acid esters.

4. Microemulsion according to any one of items 1-3, wherein the oily component (i) is selected from fatty acid triglycerides such as natural occurring oils deriving from plants or animals, fatty acid diglycerides such as diolein or dilinoleate, fatty acid monoglycerides such as monoolein or monopalmitolein, fatty acid esters of monohydric alcohol such as ethyl oleate, fatty acids such as oleic acid or linoleic acid, and mixtures thereof.

5. Microemulsion according to any of items 1-4, wherein the oily component (i) is selected from the group consisting of ethyl oleate, oleic acid, ricinus oil, corn oil, or mixtures thereof.

6. Microemulsion according to any of items 1-5, wherein the aqueous phase (ii) comprises water and optionally water soluble formulation aids, such as buffer agents, isotonic agents, viscosity-increasing compounds, antimicrobial preservatives, antioxidants, stabilizers, or mixtures thereof.

7. Microemulsion according to any of items 1-6, wherein the aqueous phase (ii) is present in an amount of 50.0 - 98.0 wt.-% based on the total weight of the microemulsion.

8. Microemulsion according to any one of items 1-7, wherein the at least one non-ionic surfactant (iii) is selected from the group consisting of alkylglucosides, polyoxyalkylene alkyl ethers, polyoxyalkylene alkyl phenols, polyoxyalkylene fatty acid esters, polyoxyalkylene glycerol fatty acid esters, polyoxyalkylene sorbitan fatty acid esters, polyoxyalkylene blockcopolymers, polyglycerol fatty acid esters, polyoxyalkylene glycerides, polyoxyalkylene sterols, polyoxyalkylene vegetable oils, polyoxyalkylene hydrogenated vegetable oils, polyglycerol ether, polyoxyalkylene glycerol ester, polyvinylalcohol, and mixtures thereof.

9. Microemulsion according to any one of items 1-8, wherein component (iii) is present in an amount of 0.40 to 10.00 wt.-% based on the total weight of the microemulsion.

10. Microemulsion according to any one of items 1-9, wherein component (iii) comprises at least two non-ionic surfactants, particularly selected from the combinations polyoxyethylene (20) sorbitan monooleate (Tween 80®) / polyoxyethylene (20) sorbitan monolaurate (Tween 20®), polyoxyethylene (20) sorbitan monooleate (Tween 80®) / polyoxyl (2) cetylether (Brij 52®), polyoxyl (2) cethylether (Brij 52®) / polyoxyl (20) cethylether (Brij 58®) and polyoxyl (20) cethylether (Brij 58®) / polyoxyl (10) cethylether (Brij 56®) as first / second non-ionic surfactant, respectively.

11. Microemulsion according to item 10, wherein the ratio of first non-ionic surfactant to second non-ionic surfactant is 10:1 - 1:1, particularly 2:1 - 1:1.

12. Microemulsion according to any one of items 1-11, wherein component (iv) is present in an amount of 0.001 to 0.100 wt.-%, preferably 0.020 to 0.050 wt.-% based on the total weight of the microemulsion.

13. Microemulsion according to any one of items 1-12, wherein component (iv) is selected from hyaluronic acid, pectin, cellulose derivatives e.g. carboxymethyl cellulose, alginic acid, carrageenan, chitosan, a pharmaceutically acceptable salt of hyaluronic acid, particularly sodium hyaluronate.

14. Microemulsion according to any one of items 1-13, wherein component (v) is present in an amount of 0.005 to 0.100 wt.-% based on the total weight of the microemulsion.

15. Microemulsion according to any one of items 1-14, wherein component (v) is capable of crosslinking component (iv).

16. Microemulsion according to any one of items 1-15, wherein component (v) has at least one carboxyl group or a salt thereof, and is preferably polycarboxylic acid e.g. citric acid, ethylenediaminetetraacetic acid (EDTA), maleic acid, a salt thereof or a mixture thereof.

17. Microemulsion according to any one of items 1-15, wherein component (v) is a cationic cross-linking agent such a multivalent metal ion, particularly $Ca^{2+}$ or $Mg^{2+}$, a salt thereof or a mixture thereof.

18. Microemulsion according to any one of items 1-17, further comprising a co-surfactant (vi).

19. Microemulsion according to item 18, wherein the co-surfactant (vi) is selected from the group consisting of non-ionic surfactants, mono alcohols, polyols and mixtures thereof.

20. Microemulsion according to any one of items 18-19, wherein the co-surfactant (vi) is present in an amount of 1.00 to 5.00 wt.-% based on the total weight of the microemulsion.

21. Microemulsion according to any one of items 1-20, wherein the weight ratio of component (iv) to component (v) is 1:1 - 1:20, preferably 1:1 - 1:2.

22. Microemulsion according to any one of items 1-21, wherein the microemulsion is an oil-in-water microemulsion.

23. Microemulsion according to any of items 1-22, wherein the mean average diameter of the oil droplets is in the range of 5 to 10,000 nm, preferably 50 to 1000 nm.

24. Microemulsion according to any of items 1-23, comprising

as component (i) at least one fatty acid ester of monohydric alcohol,
as component (ii) a pharmaceutically-acceptable water,
as component (iii) at least one polyoxyalkylene sorbitan fatty acid ester,
as component (iv) hyaluronic acid or a salt thereof,
as component (v) citric acid or a salt thereof; and
optionally as co-surfactant (vi) sorbitol.

25. Microemulsion according to item 24, wherein

component (i) is ethyl oleate,
component (iii) is a mixture of polyoxyethylene sorbitane monooleate (Tween 80®) and polyoxyethylene sorbitane monolaurate (Tween 20®), and
component (v) is calcium citrate.

26. Microemulsion according to any of items 1-25, wherein components (i) to (vi) are present in the following amounts based on the total weight of the emulsion:

1.0 - 10.0 wt.-% component (i),
50.0 - 98.0 wt.-% component (ii),
0.40 - 10.00 wt.-% component (iii),

0.001 - 0.050 wt.-%, preferably 0.020 - 0.050 wt.-% component (iv),
0.005 - 0.100 wt.-% component (v), and
optionally 1.00 - 5.00 wt.-% component (vi).

27. Microemulsion according to any of items 1-26, being free from a drug, in particular selected from rituximab, tocilizumab, rivoglitazone, mapracorat, resolving, tasoticinib, tofacitinib, voclosporin, thymosin, ecabet, rimexolone, rebamipide, diquafosol, bromfenac, dexamethasone, ciprofloxacin, ofloxacin, neomycin, tobramycin, ganciclovir, famciclovir, valganciclovir, acyclovir, hexamidine, dexamethasone, fluorometholon, hydrocortisone, prednisolone, ketorolac, diclofenac, indomethacin, ketorolac, nepafenac, bromfenac, brinzolamide, acetazolamide, dorzolamide, brimonidine, carteolol, betaxol, timolol, levobunol, latanoprost, tafluprost, bimatoprost, travoprost, atropine, cyclopentolate, tropicamid, scopolamine, tetryzoline, naphazoline, antazoline, azelastine, emedastin, levocabastin, olopatadine, ketotifen, aflibercept, ranibizumab, bevacizumab.

28. Microemulsion according to any of items 1-26, comprising at least one additional drug (vii), in particular selected from rituximab, tocilizumab, rivoglitazone, mapracorat, resolving, tasoticinib, tofacitinib, voclosporin, thymosin, ecabet, rimexolone, rebamipide, diquafosol, bromfenac, dexamethasone, ciprofloxacin, ofloxacin, neomycin, tobramycin, ganciclovir, famciclovir, valganciclovir, acyclovir, hexamidine, dexamethasone, fluorometholon, hydrocortisone, prednisolone, ketorolac, diclofenac, indomethacin, ketorolac, nepafenac, bromfenac, brinzolamide, acetazolamide, dorzolamide, brimonidine, carteolol, betaxol, timolol, levobunol, latanoprost, tafluprost, bimatoprost, travoprost, atropine, cyclopentolate, tropicamid, scopolamine, tetryzoline, naphazoline, antazoline, azelastine, emedastin, levocabastin, olopatadine, ketotifen, aflibercept, ranibizumab, bevacizumab.

29. Microemulsion according to item 28, wherein the at least one additional drug (vii) is dissolved in the oily component (i).

30. Microemulsion according to any of items 28-29, wherein component (vii) is present in an amount of 0.001-10.0 wt.-% based on the total weight of the microemulsion.

31. A process of manufacturing a microemulsion according to any of items 1-30, comprising the steps of

(a) preparing a mixture of components (i), (iii), (iv), (v), optionally (vi), and optionally (vii), and
(b) adding component (ii) to the mixture obtained in step (a) under stirring.

32. A microemulsion obtainable by a process according to item 31.

33. Pharmaceutical composition comprising a microemulsion according to any of items 1-30 or 32.

34. Pharmaceutical composition according to item 33, which is for ophthalmic applications.

35. A Microemulsion according to any of items 1-30 and 32 or pharmaceutical composition according to item 33 or 34 for use in ophthalmic applications, preferably for the prevention, alleviation and/or treatment of dry eye syndrome.

36. Use of a microemulsion according to any of items 1-30 or 32 as a carrier, in particular a drug carrier, such as an ophthalmic drug carrier.

**Claims**

1. Microemulsion comprising

    i. at least one oily component,
    ii. an aqueous phase,
    iii. at least one non-ionic surfactant,
    iv. at least one polysaccharide, a salt or a derivative thereof, and
    v. at least one cross-linking agent.

2. Microemulsion according claim 1, wherein the oily component (i) is selected from the group consisting of fatty acids and fatty acid esters, particularly unsaturated fatty acids and fatty acid esters, such as ethyl oleate, oleic acid, ricinus oil, corn oil, or mixtures thereof.

3. Microemulsion according to any one of claims 1-2, wherein the at least one non-ionic surfactant (iii) is selected from the group consisting of alkylglucosides, polyoxyalkylene alkyl ethers, polyoxyalkylene alkyl phenols, polyoxyalkylene fatty acid esters, polyoxyalkylene glycerol fatty acid esters, polyoxyalkylene sorbitan fatty acid esters, polyoxyalkylene blockcopolymers, polyglycerol fatty acid esters, polyoxyalkylene glycerides, polyoxyalkylene sterols, polyoxyalkylene vegetable oils, polyoxyalkylene hydrogenated vegetable oils, polyglycerol ether, polyoxyalkylene glycerol ester, polyvinylalcohol, and mixtures thereof.

4. Microemulsion according to any one of claims 1-3, wherein component (iv) is selected from hyaluronic acid, pectin, cellulose derivatives e.g. carboxymethyl cellulose, alginic acid, carrageenan, chitosan, preferably a pharmaceutically acceptable salt of hyaluronic acid, particularly sodium hyaluronate.

5. Microemulsion according to any one of claims 1-4, wherein component (v) is capable of crosslinking component (iv).

6. Microemulsion according to any one of claims 1-5, wherein component (v) has at least one carboxyl group or a salt thereof, and is preferably polycarboxylic acid e.g. citric acid, ethylenediaminetetraacetic acid (EDTA), maleic acid, a salt thereof or a mixture thereof.

7. Microemulsion according to any one of claims 1-6, wherein component (v) is a cationic cross-linking agent such a multivalent metal ion, particularly $Ca^{2+}$ or $Mg^{2+}$, a salt thereof or a mixture thereof.

8. Microemulsion according to any one of claims 1-7, further comprising a co-surfactant (vi), preferably selected from the group consisting of non-ionic surfactants, mono alcohols, polyols and mixtures thereof.

9. Microemulsion according to any one of claims 1-8, wherein the microemulsion is an oil-in-water microemulsion.

10. Microemulsion according to any of claims 1-9, wherein components (i) to (vi) are present in the following amounts based on the total weight of the emulsion:

    1.0 - 10.0 wt.-% component (i),
    50.0 - 98.0 wt.-% component (ii),
    0.40 - 10.00 wt.-% component (iii),
    0.001 - 0.050 wt.-%, preferably 0.020 - 0.050 wt.-% component (iv),
    0.005 - 0.100 wt.-% component (v), and
    optionally 1.00 - 5.00 wt.-% component (vi).

11. A process of manufacturing a microemulsion according to any of claims 1-10, comprising the steps of

    (a) preparing a mixture of components (i), (iii), (iv), (v), optionally (vi), and optionally (vii), and
    (b) adding component (ii) to the mixture obtained in step (a) under stirring.

12. A microemulsion obtainable by a process according to claim 11.

13. Pharmaceutical composition comprising a microemulsion according to any of claims 1-10 or 12, which is preferably for ophthalmic applications.

14. A Microemulsion according to any of claims 1-10 and 12 or pharmaceutical composition according to claim 13 for use in ophthalmic applications, preferably for the prevention, alleviation and/or treatment of dry eye syndrome.

15. Use of a microemulsion according to any of claims 1-10 or 12 as a carrier, in particular a drug carrier, such as an ophthalmic drug carrier.

**Figure 1**

**Figure 2**

**Figure 3**

NC 36h          NC 36h+6h          NC 36h+24h

DRYNESS 36h          DRYNESS 36h+6h          DRYNESS 36h+24h

P1 36h          P1 36h+6h          P1 36h+24h

P2 36h          P2 36h+6h          P2 36h+24h

P3 36h          P3 36h+6h          P3 36h+24h

P4 36h          P4 36h+6h          P4 36h+24h

**Figure 4**

| | PC 36h | P1 36h | P2 36h | P3 36h | P4 36h |
|---|---|---|---|---|---|
| ▦ AQP3 | 2,6 | 1,0 | 1,8 | 1,9 | 0,9 |
| ▦ MMP9 | 1,8 | 1,8 | 1,3 | 1,5 | 0,9 |
| ▦ MUC4 | 67,6 | 18,2 | 3,0 | 4,6 | 1,5 |
| ▦ TNF ALPHA | 1,2 | 5,0 | 1,0 | 1,7 | 1,3 |

**Figure 5**

| | PC 36h+6h | P1 36h+6h | P2 36h+6h | P3 36h+6h | P4 36h+6h |
|---|---|---|---|---|---|
| ▦ AQP3 | 3,2 | 5,3 | 2,5 | 2,7 | 2,7 |
| ▦ MMP9 | 1,7 | 1,2 | 1,1 | 1,2 | 0,7 |
| ▦ TNF ALFA | 1,9 | 8,3 | 0,7 | 2,2 | 1,1 |

**Figure 6**

| | PC 36h+24h | P1 36h+24h | P2 36h+24h | P3 36h+24h | P4 36h+24h |
|---|---|---|---|---|---|
| ▦AQP3 | 1,3 | 1,1 | 1,0 | 1,1 | 0,8 |
| ▦MMP9 | 1,5 | 1,1 | 1,0 | 1,2 | 0,5 |
| ▦TNF ALPHA | 1,1 | 2,6 | 0,6 | 0,7 | 0,5 |

**Figure 7**

| | NC | PC | P1 | P2 | P3 | P4 |
|---|---|---|---|---|---|---|
| ▦36h | 109,50 | 114,00 | 45,28 | 103,61 | 145,00 | 147,33 |

**Figure 8**

| | NC | PC | P1 | P2 | P3 | P4 |
|---|---|---|---|---|---|---|
| ▓36h+6h | 121,83 | 106,28 | 92,00 | 117,83 | 116,17 | 115,83 |
| ▓36h+24h | 149,17 | 136,06 | 57,44 | 146,33 | 146,06 | 104,72 |

**Figure 9**

Negative control 40h+24h

DRY EYE control 40h

**Figure 10**

DRY EYE control 40h+24h

AZAD Microemulsion DME04 (P1) 40h+24h

Xiidra Lifitegrast 5% (Reference) 7ED3 (P2) 40h+24h

## Figure 11

| | NC 40h+24h | PC 40h | PC 40h+24h |
|---|---|---|---|
| ▦ AQP3 | 1,00 | 2,65 | 1,92 |
| ▦ CD44 | 1,00 | 1,40 | 1,15 |
| ▦ ICAM-1 | 1,00 | 0,94 | 0,98 |
| ▦ MMP9 | 1,00 | 1,22 | 1,67 |
| ▦ TLR4 | 1,00 | 1,03 | 1,05 |
| ▦ TNFALPHA | 1,00 | 0,54 | 0,78 |

## Figure 12

| | PC 40h+24h | P1 40h+24h | P2 40h+24h |
|---|---|---|---|
| AQP3 | 1,00 | 0,45 | 0,49 |
| CD44 | 1,00 | 0,55 | 0,83 |
| ICAM-1 | 1,00 | 0,74 | 3,67 |
| MMP9 | 1,00 | 0,42 | 2,12 |
| TLR4 | 1,00 | 0,58 | 0,35 |
| TNFALPHA | 1,00 | 0,96 | 1,83 |

## Figure 13

A

**Untreated**

B

**ME5%**

C

**ME7%**

D

**Restasis®**

Europäisches
Patentamt

European
Patent Office

Office européen
des brevets

## EUROPEAN SEARCH REPORT

**Application Number**

EP 20 17 8029

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2019/036625 A1 (AKRIVISTA LLC [US]) 21 February 2019 (2019-02-21) * table 1 * | 1-4,8,9, 11-15 | INV. A61K9/00 A61K9/107 A61K47/02 |
| X | US 2015/139973 A1 (STEINFELD UTE [DE] ET AL) 21 May 2015 (2015-05-21) * paragraph [0191] * | 1-6,8,9, 11-15 | A61K47/12 A61K47/14 A61K47/26 A61K47/36 |
| X | US 2010/266710 A1 (BARONIAN MIHRAN [CH]) 21 October 2010 (2010-10-21) * example 1 * | 1-15 | A61P27/04 |
| A | JP H08 104642 A (TAKADA SEIYAKU KK) 23 April 1996 (1996-04-23) * claims 1-3 * | 1-15 | |

TECHNICAL FIELDS
SEARCHED      (IPC)

A61K
A61P

The present search report has been drawn up for all claims

| Place of search | Date of completion of the search | Examiner |
|---|---|---|
| The Hague | 12 November 2020 | Frelichowska, J |

EPO FORM 1503 03.82 (P04C01)

## ANNEX TO THE EUROPEAN SEARCH REPORT
## ON EUROPEAN PATENT APPLICATION NO.

EP 20 17 8029

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2020

| Patent document cited in search report | Publication date | Patent family member(s) | Publication date |
|---|---|---|---|
| WO 2019036625 A1 | 21-02-2019 | BR 112020003276 A2 | 01-09-2020 |
| | | CA 3072494 A1 | 21-02-2019 |
| | | CN 111295169 A | 16-06-2020 |
| | | EP 3668474 A1 | 24-06-2020 |
| | | JP 2020531462 A | 05-11-2020 |
| | | US 2020164013 A1 | 28-05-2020 |
| | | WO 2019036625 A1 | 21-02-2019 |
| US 2015139973 A1 | 21-05-2015 | AU 2013261873 A1 | 04-12-2014 |
| | | CA 2873265 A1 | 21-11-2013 |
| | | CN 104394859 A | 04-03-2015 |
| | | EP 2664329 A1 | 20-11-2013 |
| | | EP 2849741 A2 | 25-03-2015 |
| | | HK 1202427 A1 | 02-10-2015 |
| | | JP 2015521182 A | 27-07-2015 |
| | | KR 20150014966 A | 09-02-2015 |
| | | RU 2014145440 A | 10-07-2016 |
| | | US 2015139973 A1 | 21-05-2015 |
| | | WO 2013171204 A2 | 21-11-2013 |
| US 2010266710 A1 | 21-10-2010 | AU 2008280437 A1 | 29-01-2009 |
| | | CA 2694929 A1 | 29-01-2009 |
| | | CN 101778621 A | 14-07-2010 |
| | | CN 105213299 A | 06-01-2016 |
| | | EP 2178501 A2 | 28-04-2010 |
| | | ES 2671372 T3 | 06-06-2018 |
| | | JP 5872159 B2 | 01-03-2016 |
| | | JP 2010534629 A | 11-11-2010 |
| | | KR 20100051817 A | 18-05-2010 |
| | | KR 20150093244 A | 17-08-2015 |
| | | RU 2010107043 A | 10-09-2011 |
| | | US 2010266710 A1 | 21-10-2010 |
| | | WO 2009013019 A2 | 29-01-2009 |
| JP H08104642 A | 23-04-1996 | JP 3694868 B2 | 14-09-2005 |
| | | JP H08104642 A | 23-04-1996 |

EPO FORM P0459

For more details about this annex : see Official Journal of the European Patent Office, No. 12/82

**EP 3 919 047 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3409268 A
- WO 2019036625 A